# EUROPEAN PATENT SPECIFICATION

(11) **EP 1 726 248 B1**
(45) Date of publication and mention of the grant of the patent: **22.12.2010**
(21) Application number: 05720790.4
(22) Date of filing: 15.03.2005
(51) Int. Cl.: A61B 1/00

(54) **ENDOSCOPE BALLOON CONTROL DEVICE**
ENDOSKOP-BALLONKONTROLLVORRICHTUNG
DISPOSITIF DE COMMANDE DE BALLON ENDOSCOPIQUE

(30) Priority: 19.03.2004 JP 2004081654; 09.04.2004 JP 2004115847
(43) Date of publication of application: 29.11.2006
(60) Divisional application: 10009975.3
(73) Proprietor: Olympus Corporation, Tokyo 151-0072 (JP)
(72) Inventor: YOSHIDA, Takatoshi, Olympus IP Services Co. Ltd, Tokyo 192-8512 (JP); UCHIMURA, Sumihiro Olympus IP Services Co. Ltd., Tokyo 192-8512 (JP); TANIGUCHI, Akira Olympus IP Services Co., Ltd., Tokyo 192-8512 (JP)
(74) Representative: von Hellfeld, Axel
(86) International application number: PCT/JP2005/004533
(87) International publication number: WO 2005/089625

(56) References cited:
- WO-A-90/15572
- JP-A- 2002 301 019
- JP-A- 2004 337 288
- JP-A- 2005 007 030
- US-A- 5 098 379
- US-A- 5 400 770
- US-A1- 2003 083 547

## Description

### Technical Field

The present invention relates to an endoscope balloon control device which can adjust and control the amount of flow of gas sent to a balloon provided for a tip peripheral part of an inserting unit of an endoscope and a balloon provided for a tip peripheral part of an inserting unit of an overtube.

### Background Art

Generally, it is known that an endoscope is used in an alimentary canal examination. When an inserting unit of an endoscope is inserted in a deep alimentary canal, such as the small intestine in such an endoscope examination, it is difficult to insert the inserting unit into the deep part only by pushing the inserting unit in, as the intestines are complicatedly bent and it is hard to convey force to the inserting unit.

For example, when the endoscope is drawn to pick up the extra bending or slack of the endoscope which was made during the deep insertion, the tip of the inserting unit is also drawn and the bending and the slack is not picked up, which makes the deep insertion difficult.

An endoscope device adapted to prevent the tip of the inserting unit from being drawn when extra bending or slack which was made in the endoscope is picked up by attaching a balloon to the tip peripheral part of the inserting unit of the endoscope and blowing up the balloon and temporally fixing it to the intestines are proposed.

In the conventional art, an endoscope device which improves operational performance of the endoscope device by providing an overtube through which the inserting unit of the endoscope is inserted and a balloon at the tip peripheral part of the overtube and blowing up or deflating the balloon and the balloon of the endoscope as required is proposed.

For example, the Japanese Patent Laid-Open No. 2002-301019 discloses an endoscope adapted to supply air from a pump device, while control means measures an air pressure inside each of the balloon of the endoscope and the balloon of the overtube and controls a pressure inside each of the balloons.

In another conventional art, a tube with a balloon for aiding insertion of an endoscope provided with an endoscope tip balloon attached to a tip of an endoscope and a tube main body attached to the endoscope, and a tube tip balloon provided for the tip of the tube body as shown in the Japanese Patent Laid-Open No. 2001-340462.

The invention disclosed in the abovementioned Patent Gazette is such that a balloon is attached to each of an endoscope and a tube to be attached to the endoscope and inserting performance of the endoscope into a living body and operability of the endoscope in a living body is improved with the balloon.

In the endoscope device and the tube with a balloon for aiding insertion of an endoscope disclosed in the Patent Gazette, balloons are differently blown up even if they have the same amount of flow, if the balloon of the endoscope and the balloon of the overtube are made of different materials or materials of the balloons differ each time the balloons are exchanged. Accordingly, even if the material or the type of the balloons differs, the amount of gas flow for each of the balloons needs to be controlled according to the material of the balloon to keep the balloon to a constant way of blowing.

The balloons are formed of various materials and have various capacities, and are used depending on the treatment, the type of the endoscope used for the treatment, and individual difference of living bodies. Therefore, for example, in a site where a treatment is performed to a living body, the treatment may be performed while balloons are exchanged for multiple times. In such a case, the conventional example has a problem that an operator or an assistant or the like of the operator need to check possibility of poor fixing in a living body occurring to a balloon to be exchanged each time balloons are exchanged, which increases a treatment time to the living body.

Document US 5,400,770 concerns a surgical device for use with an endoscope having an insertion member insertable into a patient. The device comprises an inflatable balloon, an attachment component for removably attaching the balloon in a collapsed configuration to the endoscope Insertion member and an inflation device connected to the balloon for enabling an expansion of the balloon from the collapsed configuration to an expanded configuration.

Document US 5,098,379 concerns a catheter comprising a balloon portion. In order to test that there are no leaks In the balloon portion, a proximal capillary lumen accessing opening is engaged to the nozzle of a commercially available leak tester

Document JP 2002-301019 concerns an endoscope device including an endoscope body having a body-fixing balloon mounted on the outer periphery of its tip, a sliding tube having a tube-fixing balloon mounted on the outer periphery of its tip, and pump devices for supplying air to the balloons. The pump devices have control means for controlling pressures within the balloons by measuring the pressures of air in the balloons.

The present invention is adapted in view of the circumstances, and intends to provide an endoscope balloon control device which can keep a constant way of blowing up even if the materials and the type of the balloons are different by controlling the amount of flow of gas sent or drawn to a balloon of an endoscope and a balloon of an overtube.

The present invention intends to provide an endoscope balloon control device and an abnormality determining method of the endoscope balloon control device which can simply and automatically determine abnormality of a balloon duct when it controls the amount of flow of gas sent or drawn to the balloon.

### Disclosure of the Invention

The present invention concerns an endoscope balloon control device having the features of claim 1.

In order to achieve the abovementioned objects, the endoscope balloon control device of the present invention is as defined in the appended claims.

### Brief Description of the Drawings

Figure 1 is a configuration diagram showing an entire configuration of an endoscope system to which an endoscope balloon control device is applied according to a first embodiment of the present invention;
Figure 2 is a configuration diagram showing an outlined configuration of the endoscope balloon control device of Figure 1;
Figure 3 is a diagram showing a configuration example of the remote controller of Figure 2;
Figure 4 is a block diagram showing an inner configuration of the endoscope balloon control device of Figure 2;
Figure 5 is an illustration showing a state where each balloon is deflated and an endoscope is inserted through an overtube and inserted into the intestines;
Figure 6 is an illustration showing a state where a balloon for an overtube is blown up and fixed in the intestines;
Figure 7 is an illustration showing a state where the endoscope is further inserted into the overtube from the state of Figure 6;
Figure 8 is an illustration showing a state where the balloon of the endoscope is blown up and fixed to the wall of the intestines in the state of Figure 7;
Figure 9 is an illustration showing a state where the balloon of the overtube is deflated and the overtube is further inserted in the state of Figure 8;
Figure 10 is an illustration showing a state where the tip of the overtube moves to the tip part of the endoscope from the state of Figure 9;
Figure 11 is an illustration showing a state where the balloon of the overtube is blown up and fixed to the wall of the intestines in the state of Figure 10;
Figure 12 is a flowchart showing a main program of a control unit for illustrating an operation of an endoscope balloon control device;
Figure 13 is a flowchart showing a processing routine based on the switch state checking module of Figure 12;
Figure 14 is a flowchart showing a processing routine based on the gas sending module of Figure 13;
Figure 15 is a flowchart for showing a processing routine based on the gas drawing module of Figure 13;
Figure 16 is a configuration diagram showing an outlined configuration of an endoscope balloon control device according to a second embodiment of the present invention;
Figure 17 is a block diagram showing an inner configuration of the endoscope balloon control device of Figure 16;
Figure 18 is a flowchart showing control contents of an endoscope balloon control device according to the second embodiment;
Figure 19 is a graph showing relationship between an inner pressure of a balloon for an endoscope or a balloon for an overtube and a gas sending time when it is determined as abnormal adjustment ending information in the minimum amount of flow setting of the pump of Figure 18;
Figure 20 is a graph showing relationship between an inner pressure of a balloon for an endoscope or a balloon for an overtube and a gas sending time when it is determined as abnormal adjustment ending information in the maximum amount of flow setting of the pump of Figure 18;
Figure 21 is a graph showing relationship between an inner pressure of a balloon for an endoscope and a balloon for an overtube and a gas sending time when abnormality is not recognized even if the amount of flow setting of the pump of Figure 18 is changed from the minimum amount of flow setting to the maximum amount of flow setting;
Figure 22 is a graph showing relationship between an inner pressure of a balloon for an endoscope or a balloon for an overtube and a gas sending time when a change in an inner pressure of duct is checked by using an inner pressure of a balloon for an endoscope or a balloon for an overtube as a predetermined inner value to be an almost equilibrium state after the amount of flow setting of the pump of Figure 18 is made the amount of flow set after adjustment; and
Figure 23 is a configuration diagram where a test jig which substitutes for a balloon for an endoscope and a balloon for an overtube and an adjustment processor are attached to an endoscope balloon control device for examination executed by using an endoscope balloon control device.

### Best Mode for Carrying Out the Invention

Embodiments of the present invention will be described with reference to the drawings.

### (Embodiment 1)

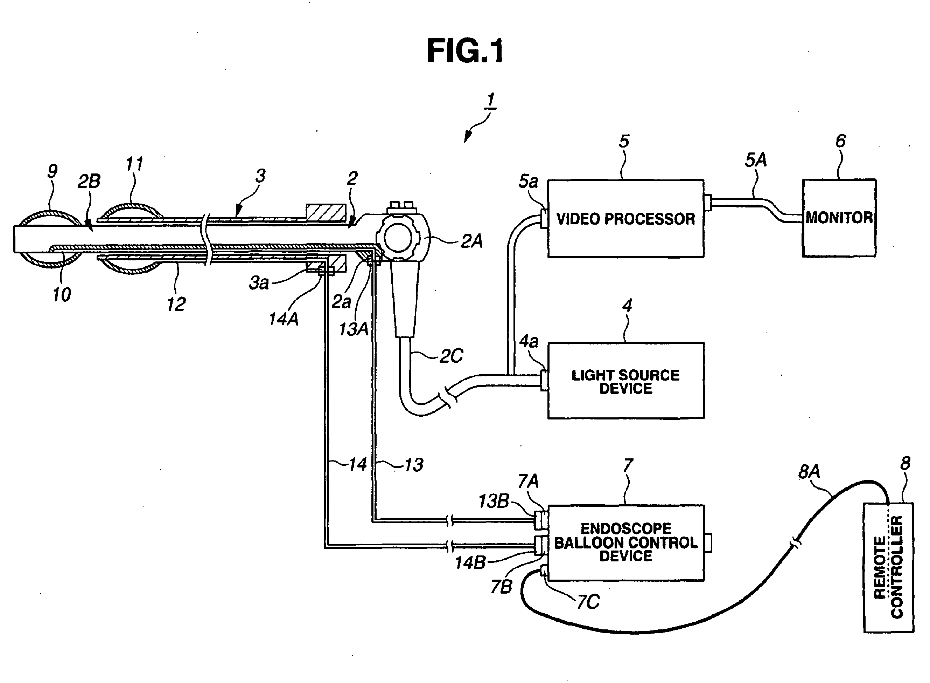

Figure 1 is a configuration diagram showing an entire configuration of an endoscope system applied with an endoscope balloon control device according to a first embodiment of the present invention.

As shown in Figure 1, an endoscope system 1 having an endoscope balloon control device of the embodiment has an endoscope 2, an overtube 3, a light source device 4, a video processor 5, a monitor 6, an endoscope balloon control device 7 and a remote controller 8.

The endoscope 2 is used for an alimentary canal endoscope examination, for example, having an inserting unit 2B to be inserted in a body cavity and an operation unit 2A provided at a rear anchor side of the inserting unit 2B. An observation optical system including an illumination optical system and a CCD which is an image pickup device (not shown) is provided in the tip part of the inserting unit 2B and illuminates an observed part in the alimentary canal of a subject body and can obtain an observation image in the alimentary canal of the subject body.

A universal code 2C is extending out in the operation unit 2A. Inside the universal code 2C, a signal line and a light guide cable (not shown) are provided. The base end part of the universal code 2C is connected to a connector 4a of the light source device 4 and a connector 5a of the video processor 5. Accordingly, illumination light from the light source device 4 is supplied to the illumination optical system of the endoscope 2 via the light guide cable in the universal code 2C and illuminates the observed part, and outputs an image pick up signal in the alimentary canal outputted from the CCD to the video processor 5.

Such an endoscope 2 is adapted to be used inserted through the overtube 3 during an operation. A configuration of the overtube 3 will be described later.

The light source device 4 is a light source device for supplying illumination light to the illumination optical system provided for the endoscope 2 via a light guide (not shown) in the light guide cable.

The light source device 4 has a connector 4a outside and the connector 4a has a configuration to be detachably connected to a universal code 2C of the endoscope 2.

The video processor 5 has a configuration to be detachably connected to a connecting cable branched from the universal code 2C. The video processor 5 performs signal processing on an image pickup signal from the CCD of the endoscope 2 and supplies image data based on the image pickup signal (for example, endoscope live image data) to the monitor 6.

The monitor 6 is connected to the video processor 5 by a connecting cable 5A. The monitor 6 displays an endoscope image based on image data from the video processor 5.

In the endoscope system 1 of the embodiment, a fixing balloon for an endoscope 9 (hereinafter referred to as a balloon 9) is attached to the tip peripheral part of the inserting unit 2B of the endoscope 2. An air supplying tube 10 provided along the inserting unit 2B from the base end part side to the tip part side of the inserting unit 2B is connected to the balloon 9.

The base end part of the operation unit 2A of the air supplying tube 10 is connected to the connector 2a provided at the bottom of the operation unit 2A. To the connector 2a is connected a connector 13A which is provided at one end of an endoscope balloon gas sending tube (hereinafter, referred to as the first gas sending tube) 13, the other end of which is connected to an endoscope balloon control device 7 to be described later.

The first gas sending tube 13 has the connector 13A at one end and a connector 13B at the other end. The connector 13B at the other end of the first gas sending tube 13 is connected to a connector 7A of the endoscope balloon control device 7.

With the connection of the connector 13B and the connector 7A, it has a structure to keep air sealing inside the first gas sending tube 13. Further, similarly, with the connection of the connector 2a and the connector 13A, it has a structure to keep air sealing inside the air supplying tube 10 and the first gas sending tube 13.

With the configuration, the balloon 9 is blown up by gas sent from the endoscope balloon control device 7 and can be temporally fixed to the alimentary canal such as the intestinal canal.

The overtube 3 is for guiding the inserting unit 2B to be inserted in the alimentary canal, for example, by inserting the endoscope 2 through it, and has a little bit bigger inside diameter than the external diameter of the inserting unit 2B of the endoscope. The overtube 3 has a configuration with the same flexibility as the inserting unit 2B of the endoscope 2. Further, to the tip peripheral part of the overtube 3, a tube fixing balloon for an overtube (hereinafter referred to as a balloon 11) is attached.

To the balloon 11, an air supplying tube 12 provided for the overtube 3 from its base end part side to its tip part side is connected.

The base end part at the opposite side of the balloon 11 of the air supplying tube 12 (an inserting slot side for inserting the endoscope 2 of the overtube 3) is connected to a connector 3a provided near the inserting slot of the overtube 3. To the connector 3a is connected a connector 14A which is provided at one end of an overtube balloon gas sending tube (hereinafter referred to as a second gas sending tube) 14, the other end of which is connected to the endoscope balloon control device 7.

The second gas sending tube 14 has the connector 14A on one end and a connector 14B on the other end. The connector 14B on the other end of the second gas sending tube 14 is connected to a connector 7B of the endoscope balloon control device 7B.

With the connection of the connector 14B and the connector 7B, it has a structure to keep air sealing inside the second gas sending tube 14. Further, similarly, with the connection of the connector 3a and the connector 14A, it has a structure to keep air sealing inside the air supplying tube 12 and the second gas sending tube 14.

With the configuration, the balloon 11 is blown up by gas sent from the endoscope balloon control device 7 and can be temporally fixed to the alimentary canal such as the intestine canal.

The endoscope balloon control device 7 is for controlling various operations on the amount of flow of gas sent to the balloon 9 of the endoscope 2 and the balloon 11 of the overtube 3 and the like.

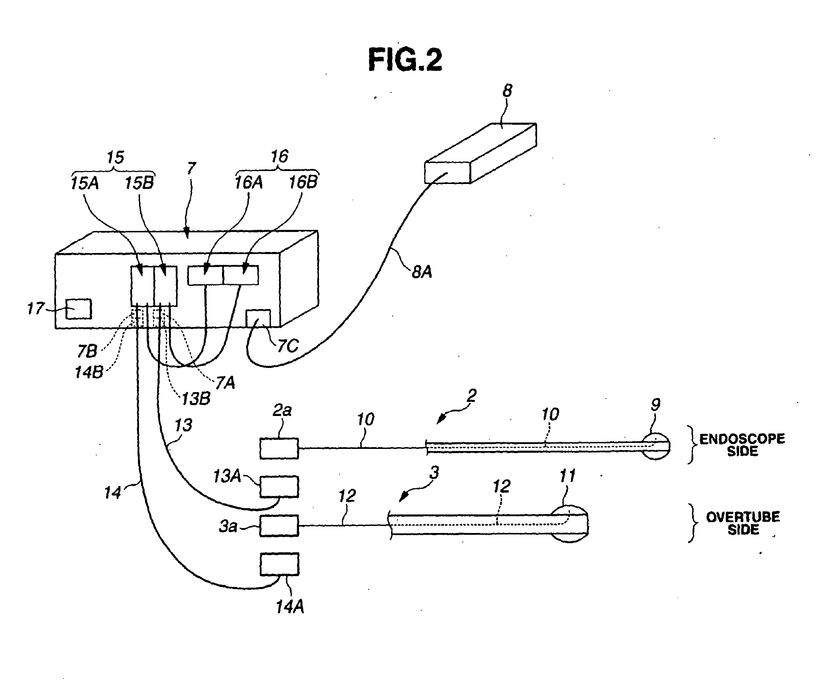

Figure 2 is a configuration diagram showing an outlined configuration of the endoscope balloon control device.

As shown in Figure 2, a backflow prevention tank 15 is provided for the endoscope balloon control device 7, and a pressure display device 16 and a power source switch 17 are provided at the front side of the device 7.

The backflow prevention tank 15 is adapted to be able to prevent backflow of a liquid, having a tank 15A for the balloon 9 of the endoscope 2 and a tank 15B for the balloon 11 of the overtube 3. The first and the second gas sending tubes 13 and 14 are connected to the corresponding tanks 15A and 15B respectively.

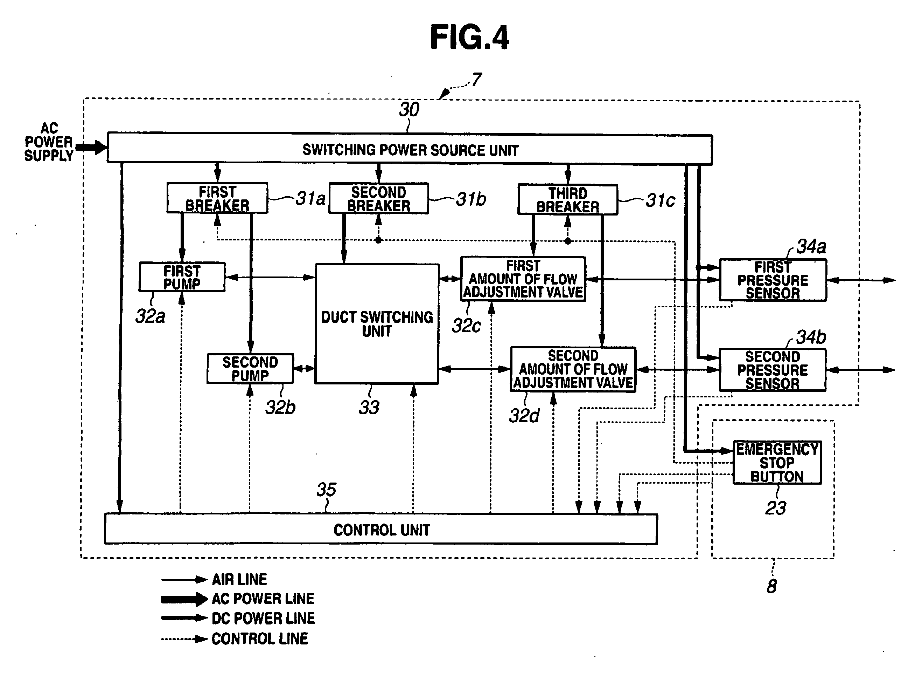

The tanks 15A and 15B send gas to the balloons 9 and 11 respectively via the first and the second gas sending tubes 13 and 14 by increasing the inside pressure via the first and the second pumps 32a and 32b (see Figure 4) to be described later by controlling the endoscope balloon control device 7, respectively. In such a case, the tanks 15A and 15B are adapted to prevent backflow of a liquid from the first and the second gas sending tubes 13 and 14 by a backflow prevention mechanism (not shown).

As such, a gas sending duct via the air supplying tube 10 connected to the balloon 9 of the endoscope 2, the first gas sending tube 13, and the tank 15B, a gas sending duct via an air supplying tube 12 connected to the balloon 11 of the overtube 3, the second gas sending tube 14, and the tank 15A are provided for the endoscope balloon control device 7 of the embodiment.

The pressure display device 16 is for displaying a pressure value of ducts connected to the balloons 9 and 11 by using a detecting device (not shown). The pressure display device 16 has a display device 16A for the balloon 9 of the endoscope 2 and a display device 16B for the balloon 11 of the overtube 3.

The display device 16A displays a pressure value inside the duct for the balloon 9 of the endoscope 2, and the display device 16B displays a pressure value inside the duct for the balloon 11 of the overtube 3.

The power source switch 17 is a switch for switching the power source of the endoscope balloon control device 7 between ON and OFF.

As shown in Figure 1 and Figure 2, the remote controller 8 is connected to a side of the endoscope balloon control device 7 via a connecting cable 8A. The remote controller 8 is electrically connected to a control unit 35 provided inside the endoscope balloon control device 7 to be described later via the connecting cable 8A.

In the embodiment, the endoscope balloon control device 7 is adapted to be supplied with an operation signal for pressure control and gas sending control of each of the balloons 9 and 11 by an operator to manipulate the remote controller 8 during an operation.

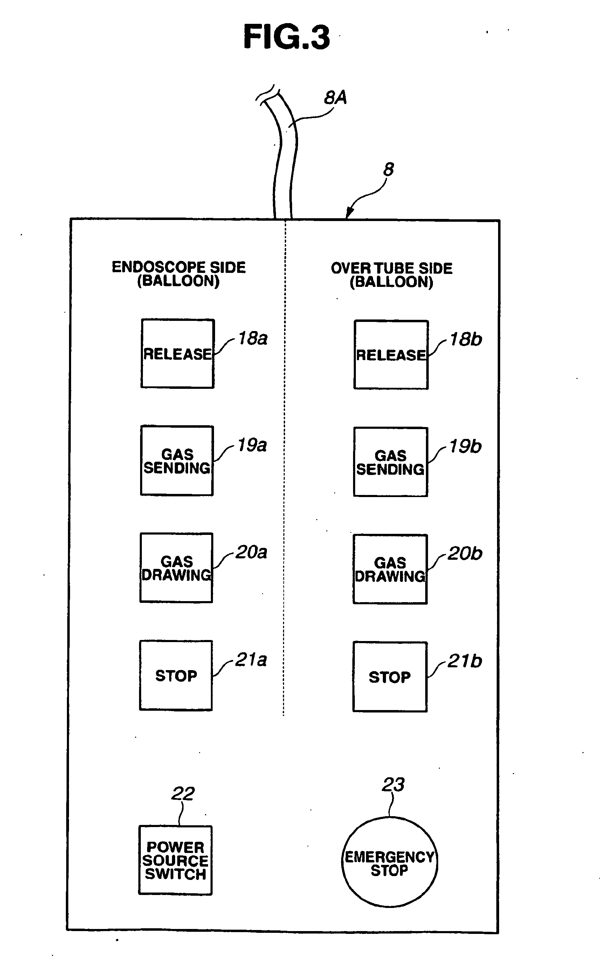

Figure 3 is a diagram showing a configuration example of the remote controller 8.

As shown in Figure 3, the remote controller 8 is set up as its various buttons for controlling an endoscope side balloon and various buttons for controlling an overtube side balloon are divided into right and left of the main body of the remote controller, for example, for facilitating manipulation of the operator.

At the left side of the remote controller 8, a release button 18a, a gas sending start button 19a, a gas drawing start button 20a and a stop button 21 a are provided as manipulation buttons for controlling the endoscope side balloon.

To the right side of the remote controller 8, a release button 18b, a gas sending start button 19b, a gas drawing start button 20b and a stop button 21b are provided as manipulation buttons for controlling the overtube side balloon.

Further, a power source button 22, and an emergency stop button 23 are provided at the bottom of the remote controller 8.

The release button 18a is a button for indicating to release air in a duct of the balloon 9 of the endoscope 2. The gas sending start button 19a is a button for indicting to start sending gas into the balloon 9 of the endoscope 2. The gas drawing start button 20a is a button for indicating to start drawing gas from the balloon 9 of the endoscope 2. The stop button 21a is a button for performing indication to stop a gas sending operation by the gas sending start button 19a or a gas drawing operation by the gas drawing start button 20a. As the stop button 21a is pressed down, an air pressure inside the balloon 9 can be kept.

On the other hand, the release button 18b is a button for indicating to release air inside the duct of the balloon 11 of the overtube 3. The gas sending start button 19b is a button for indicating to start sending gas into the balloon 11 of the overtube 3. The gas drawing start button 20b is a button for indicating to start drawing gas from the balloon 11 of the overtube 3. The stop button 21b is a button for performing indication to stop a gas sending operation by the gas sending start button 19b and a gas drawing operation by the gas drawing start button 20b. As the stop button 21b is pressed down, an air pressure inside the balloon 11 or a duct can be kept.

The power source button 22 is a button for switching the power source of the endoscope balloon control device 7 between the ON state and the OFF state.

The emergency stop button 23 is a button for directly turning off the first to the third breaker 31 a to 31c to be described later of the endoscope balloon control device 7 and perform emergency stop on gas sending control or the like of each of the balloons 9 and 11 by the endoscope balloon control device 7.

Next, an inner configuration of the endoscope balloon control device 7 will be described with reference to Figure 4. Figure 4 is a block diagram showing an inner configuration of the endoscope balloon control device.

As shown in Figure 4, the endoscope balloon control device 7 has a switching power source unit 30, a first to a third breakers 31a to 31c, a first and a second pumps 32a, 32b, a first and a second amount of flow adjustment valves 32c, 32d, a duct switching unit 33, a first and a second pressure sensors 34a, 34b, and a control unit (control unit) 35 having the abnormality determining section.

To the switching power source unit 30, AC power is supplied from an external commercial power source via a connecting code (not shown). The switching power source unit 30 converts the supplied AC power into DC power and supplies it to the first to the third breakers 31a to 31c, the first and the second pressure sensors 34a and 34b, the control unit 35 and the emergency stop button 23 of the remote controller 8.

The first breaker 31a is electrically connected to the first and the second pumps 32a and 32b and the emergency stop button 23 of the remote controller 8. The first breaker 31a supplies DC power to the first and the second pumps 32a and 32b. If an operation signal is supplied from the emergency stop button 23, supplying of DC power to the first and the second pumps 32a and 32b is stopped.

The second breaker 31b is electrically connected to the duct switching unit 33 and the emergency stop button 23 of the remote controller 8. The second breaker 31b supplies DC power to the duct switching unit 33. If an operation signal is supplied from the emergency stop button 23, supplying of DC power to the duct switching unit 33 is stopped.

The third breaker 31c is electrically connected to the first and the second amount of flow adjustment valves 32c and 32d and the emergency stop button 23 of the remote controller 8. The third breaker 31c supplies DC power to the first and the second amount of flow adjustment valves 32c and 32d. If an operation signal is supplied from the emergency stop button 23, supplying of DC power to the first and the second amount of flow adjustment valves 32c and 32d is stopped.

The first and the second pumps 32a and 32b are connected to an input side of the duct switching unit 33 via an air line, respectively. The first and the second pumps 32a and 32b are adapted to be controlled for driving based on a control signal from the control unit 35. For example, they are adapted to send air, which is gas, to the duct switching unit 33 via the air line, or, on the contrary, to draw air from the duct switching unit 33 via the air line.

To the output side of the duct switching unit 33, the first and the second amount of flow adjustment valves 32c and 32d are connected via an air line, respectively. The first and the second amount of flow adjustment valves 32c and 32d are valves which can be adjusted between open and close by the control unit 35, and makes adjustment of the amount of flow of air to be output based on a control signal from the control unit 35. The first and the second amount of flow adjustment valves 32c and 32d supply air by the adjusted amount flow within a predetermined range to the first and the second pressure sensors 34a and 34b via a gas sending line, respectively.

The fist and second pressure sensors 34a and 34b measure air pressures supplied from the first and the second amount of flow adjustment valves 32c and 32d. In the embodiment, measured results by the first and the second pressure sensors 34a and 34b may be supplied to the control unit 35 and the control unit 35 may be adapted to control the first and the second pumps 32a and 32b to make them desired air pressures based on the supplied measured results respectively.

Outputs from the first and the second pressure sensors 34a and 34b are adapted to be supplied to the first and the second gas sending tubes 13 and 14 via a gas sending line, connectors 7A, 7B, 13B and 14B, respectively.

As such, the endoscope balloon control device 7 has a gas sending duct comprising the first amount of flow adjustment valve 32c and the first pressure sensor 34a via the fist pump 32a and the duct switching unit 33, and a gas sending duct comprising the second amount of flow adjustment valve 32d and the second pressure sensor 34b via the second pump 32b and the duct switching unit 33.

The duct switching unit 33 can switch ducts (not shown) provided inside so as to make it a duct state according to an executing mode in the endoscope balloon control device 7. For example, as the executing mode, there are four executing modes such as a gas sending mode, a gas drawing mode, a keeping mode, and a releasing mode. Therefore, the duct switching unit 33 can switch an inner duct (not shown) to make it a state according to the four modes, i.e., a gas sending state, a gas drawing state, a keeping state, and a releasing state. The switching can be controlled based on a control signal from the control unit 35. As a result, it is adapted to make the duct at the balloon 9 side of the endoscope 2 and the duct at the balloon 11 side of the overtube 3 connected to the back side duct states based on respective desired executing modes.

Although they are not shown, the control unit 35 has an amount of flow counter, which is the amount of flow detecting section for counting the amount of flow of gas sent to the balloon 9 of the endoscope 2 and the balloon 11 of the overtube 3; a timer counter for counting each of a gas sending time and a gas drawing time of the balloons 9 and 11 or a timer for measuring a predetermined time; and a storing unit storing a main program to be described later or a program based on various modules.

The control unit 35 is adapted to control the first and the second pumps 32a and 32b, and the duct switching unit 33 and the first and the second amount of flow adjustment valves 32c, 32d by using the amount of flow counter and the timer counter by executing the program based on an operation signal from the remote controller 8.

Then, the endoscope balloon control device 7 is adapted to be able to measure a gas sending time or a gas drawing time to or from the balloon 9 of the endoscope 2 and the balloon 11 of the overtube 3, and time of the amount of flow of gas sent and the like, and control the amount of flow of gas sent to the balloon 9 of the endoscope 2 and the balloon 11 of the overtube 3 by using the measured result.

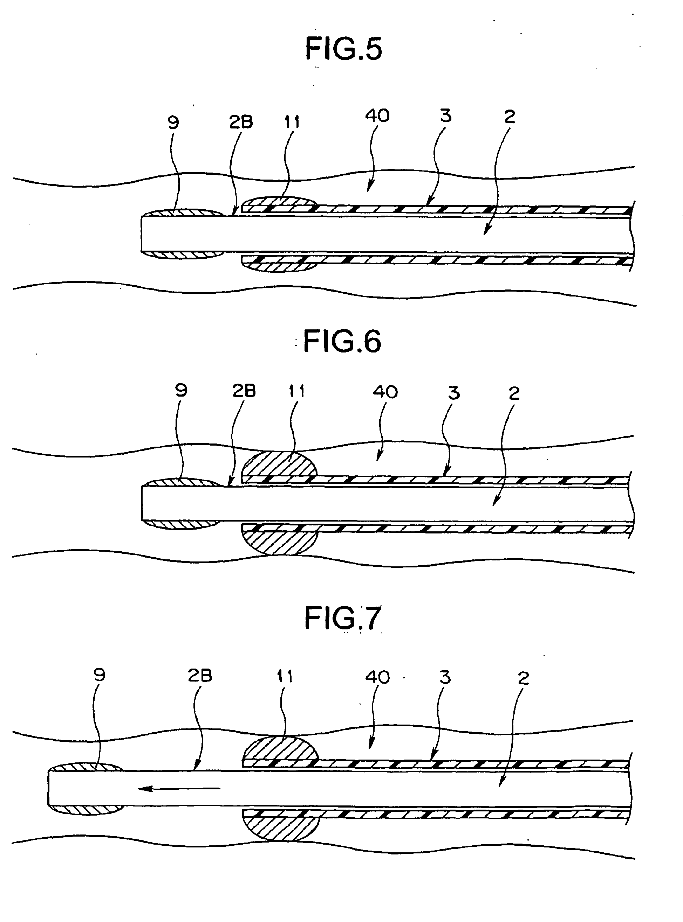

Next, a basic operation state of the endoscope system 1 will be described with reference to Figure 5 to Figure 11.

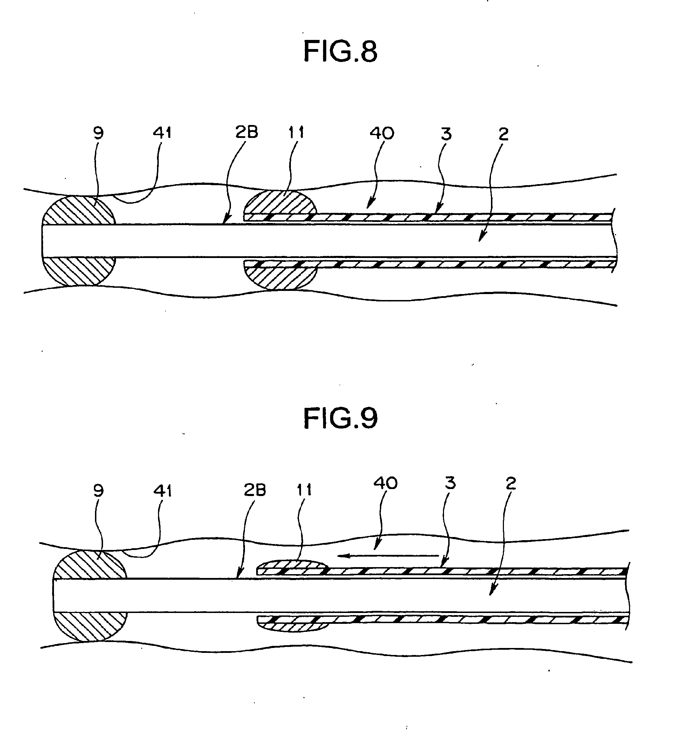

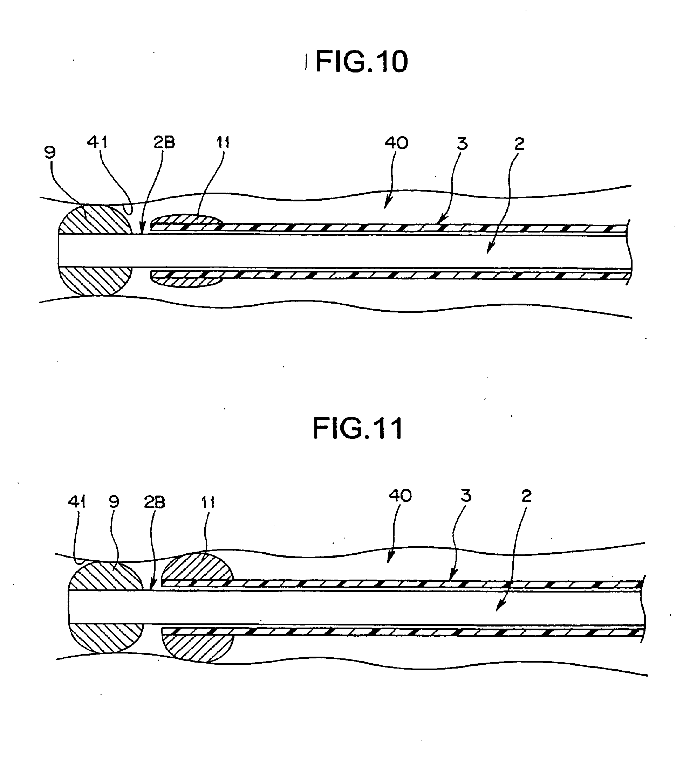

From Figure 5 to Figure 11 are illustrations describing an operation state of an endoscope and an overtube by using the balloon of the endoscope and the balloon of the overtube. Figure 5 shows a state where each balloon is deflated and an endoscope is inserted through the overtube and inserted into the intestines; Figure 6 shows a state where the balloon of the overtube is blown up and fixed to the intestines; Figure 7 is a state where the endoscope is further inserted into the overtube from the state of Figure 6; Figure 8 shows a state where the balloon of the endoscope is blown up and fixed to the intestine wall in the state of Figure 7; Figure 9 shows a state where the balloon of the overtube is deflated and the overtube is further inserted in the state of Figure 8; Figure 10 shows a state where the tip of the overtube moves to the endoscope tip from the state of Figure 9; and Figure 11 shows a state where the balloon of the overtube is blown up and fixed to the intestine wall in the state of Figure 10, respectively.

As shown in Figure 5, an operator inserts the endoscope 2 into the overtube 3. In such a case, the balloon 9 of the endoscope 2 and the balloon 11 of the overtube 3 are in a deflated state as air inside are drawn respectively, and the operator starts insertion of the endoscope 2 to the subject in this state.

Next, when the operator inserted the tip of the endoscope 2 and the overtube 3 to, for example, a descending leg of duodenum, the operator presses down the gas sending start button 19b (see Figure 3) at the overtube side of the remote controller 8 to supply air into the balloon 11 for fixing the main body attached to the tip of the overtube 3 from the second pump 32b, blow up the balloon 11 and fix the overtube 3 to the intestines 40 as shown in Figure 6.

Next, the operator keeps the overtube 3 to the intestines 40 and inserts only the inserting unit 2B of the endoscope 2 into the deep part as shown in Figure 7.

Then, the operator presses down the gas sending start button 19a (see Figure 3) at the endoscope side of the remote controller 8 in a state where the inserting unit 2B of the endoscope 2 is inserted by a predetermined distance to supply air into the balloon 9 for fixing the main body attached to the tip of the endoscope 2 from the first pump 32a, blow up the balloon 9 and fix to the intestine wall 41 as shown in Figure 8.

Some patients may have narrow intestines 40, however, the operator can optimally blow up each of the balloons 9 and 11 and fix it to the intestines 40 according to the size of the intestines 40 only by pressing down the stop buttons 2 1 a and 21b to stop air supply to each of the balloons 9 and 11.

Next, the operator presses down the release button 18b or the gas drawing start button 20b (see Figure 3) at the overtube side of the remote controller 8 to release air in the balloon 11 by the duct switching unit 33 or draw air in the balloon 11 of the overtube 3 from the second pump 32b and deflate the balloon 11 (see Figure 9).

Next, the operator inserts the overtube 3 to the deep part along the endoscope 2 and inserts the tip of the overtube 3 near the tip of the inserting unit 2B of the endoscope 2 as shown in Figure 9.

Then, the operator presses down the gas sending start button 19b (see Figure 3) at the overtube side of the remote controller 8 in the state where the tip of the overtube 3 is inserted near the tip of the inserting section 2B to supply air to the balloon 11 of the overtube 3 from the second pump 32b, blow up the balloon 11 and fix the overtube 3 to the intestines wall, as shown in Figure 11.

The operator presses down the release button 18a or the gas drawing start button 20a at the endoscope side of the remote controller 8 (see Figure 3) to release air in the balloon 9 by the duct switching unit 33 or draw air in the balloon 9 of the endoscope 2 from the first pump 32a and deflate the balloon 9 and further insert the inserting unit 2B to the deep part.

As the operations from Figure 5 to Figure 11 are repeated, insertion of the endoscope 2 and the overtube 3 into the deep part is advanced, so that the inserting unit 2B of the endoscope 2 can be inserted to a desired place.

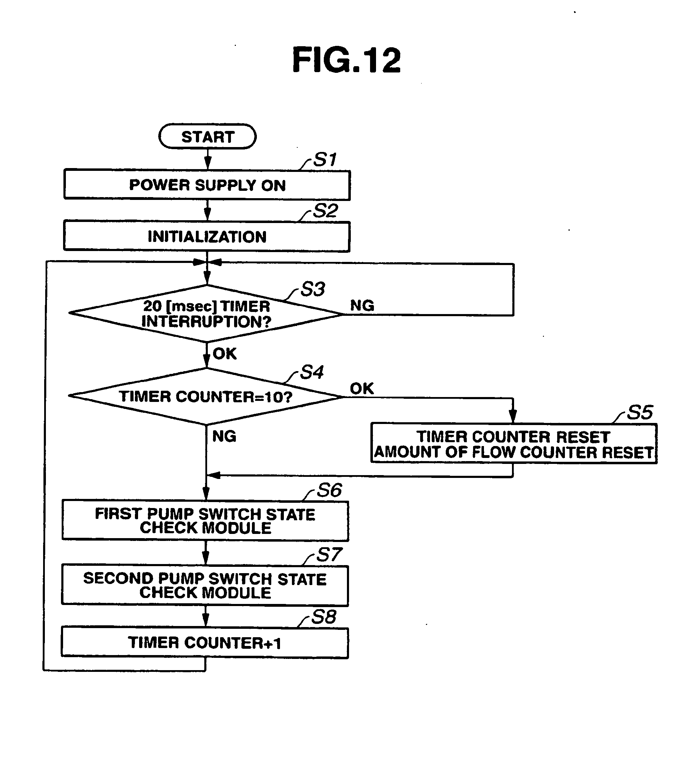

Next, operations of the endoscope balloon control device of the embodiment will be described with reference to Figure 12 to Figure 15.

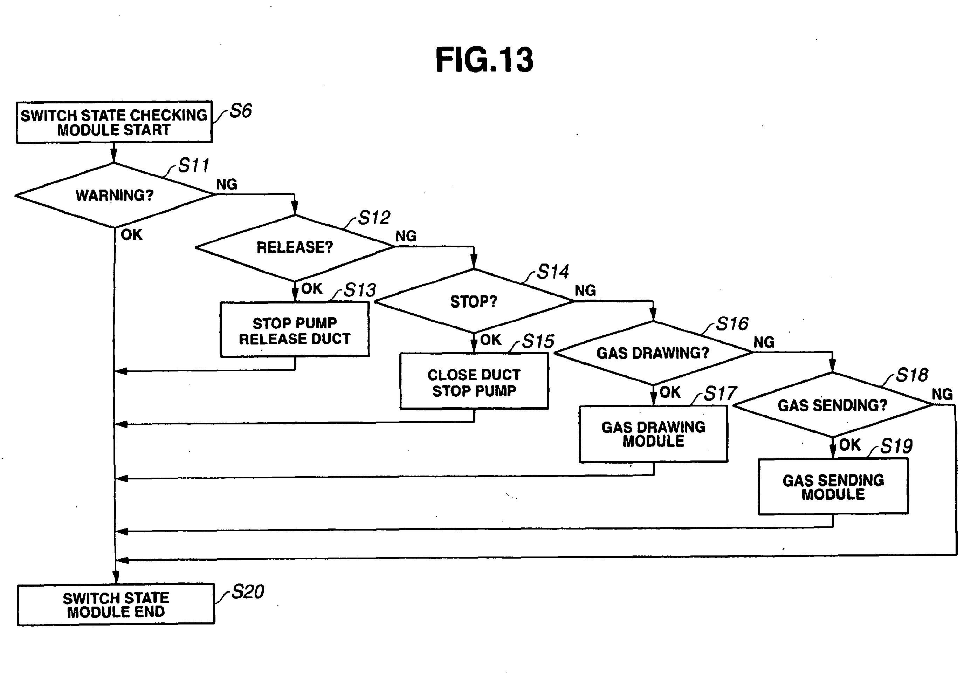

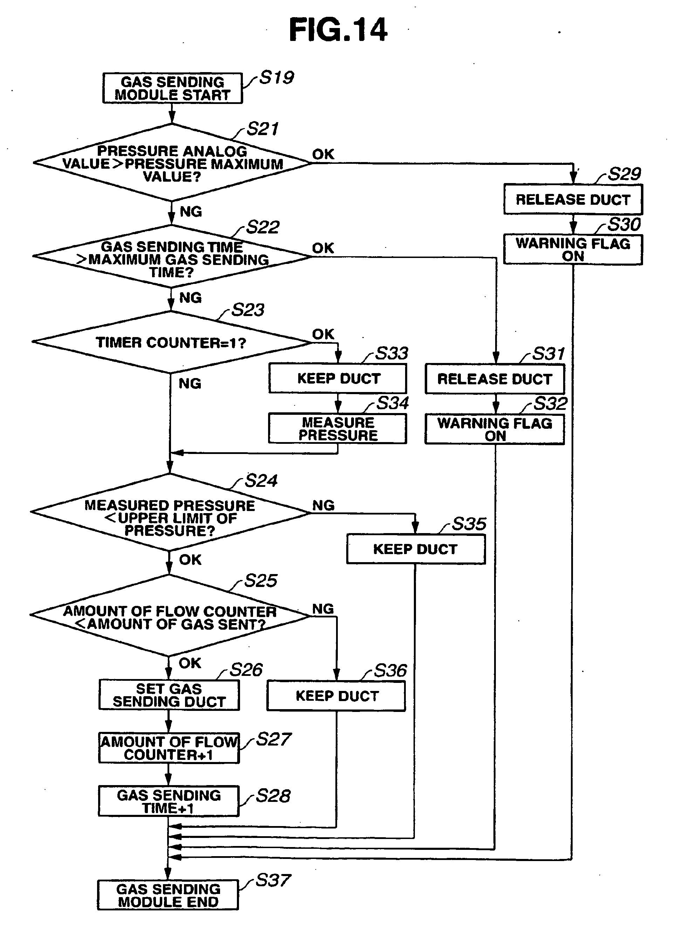

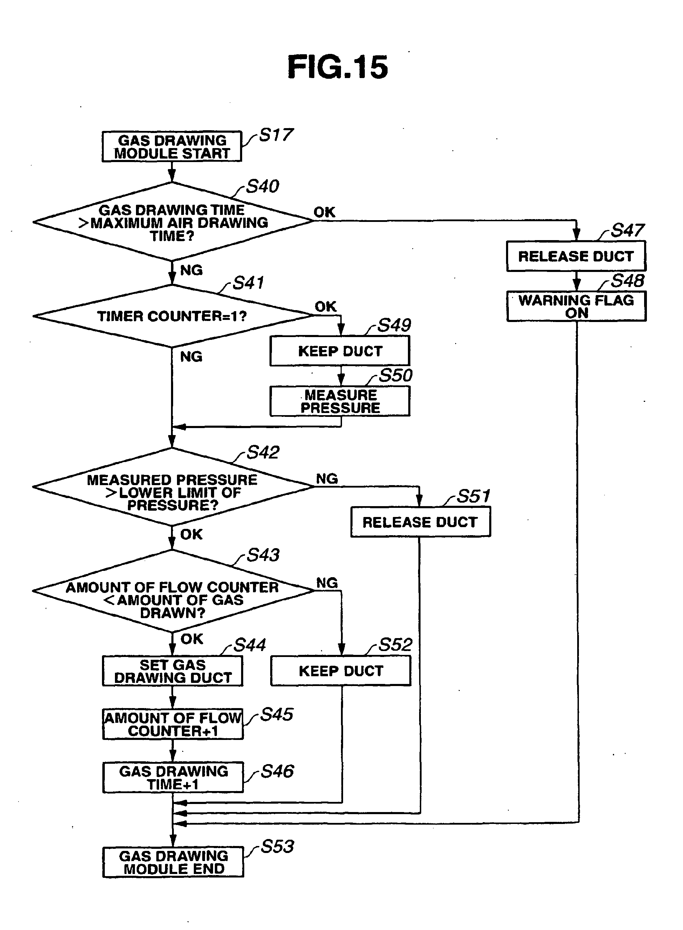

From Figure 12 to Figure 15 are for describing operations of the endoscope balloon control device and; Figure 12 is a flowchart for showing a main program of a control unit; Figure 13 is a flowchart for showing a processing routine based on a switch state checking module of Figure 12; Figure 14 is a flowchart for showing a processing routine based on a gas sending module of Figure 13; and Figure 15 is a flowchart for showing a processing routine based on a gas drawing module of Figure 13.

It is assumed that an operator performs an endoscope examination in the alimentary canal by using the endoscope system 1 of Figure 1. When the operator presses down a power source button 22 of the remote controller 8 shown in Figure 3 (or a power source switch 17 shown in Figure 2), the control unit 35 reads in a main program shown in Figure 12 from a storing unit (not shown) inside and starts it.

When the control unit 35 checks the ON state of the power source by the process at the step S1, it performs initialization on various appliances and the like in the endoscope balloon control device 7 by the process at the step S2. As the initialization, for example, the control unit 35 starts the first and the second pumps 32a and 32b and performs initialization so that it is in a duct release state by the duct switching unit 33. The control unit 35 initializes by resetting an amount of flow counter, a timer count and the like (not shown) in the control unit 35.

Then, the control unit 35 determines 20 msec timer interruption by the determination process at the following step S3; and if it determines that it occurred, it transfers the process to the step S4; and if it determines that it did not occur, it keeps performing the determination process.

For the timer, one for measuring 20 msec is used for operating the processing routine by every 20 msec shown in Figure 12.

Then, the control unit 35 determines whether the counter value of the timer counter for counting one by every 20 msec of the timer is equal to 10 or not by the determination process at the step S4; and if it determines that they are equal, it resets the timer counter and the amount of flow counter by the process at the step S5 and transfers the process to the step S6. On the other hand, if it determines that the counter value is not equal to 10, the control unit 35 transfers the process to the step S6.

In the embodiment, it is assumed that balloon control is performed according to manipulation of various buttons by the operator using the controller 8 by ten times of the processing routine shown in Figure 12, i.e., by the time unit of 200 msec.

Next, the control unit 35 executes the processing routine based on the first pump and the second pump switch state checking module to be described later by the processes at the step S6 and the step S7.

Then, after the processing routine based on the switch state checking module has been performed, the control unit 35 adds 1 to the counter value by the timer counter at the process at the step S8 and then returns the process to the determination process at the step S3.

Next, the processing routine of the first pump switch state checking module by the step S6 will be described with reference to Figure 13.

When the control unit 35 executes the process at the step S6, it starts the processing routine of the switch state checking module shown in Figure 13.

Then, the control unit 35 determines the presence of warning by whether the emergency stop button 23 of the remote controller 8 has been pressed down or not by the determination process at the step S 11. In such a case, the control unit 3 5 performs the determination process by detecting ON/OFF of the warning flag. The warning flag is adapted to be turned ON not only by the emergency stop button 23 being pressed down but also by the control unit 35 when the endoscope balloon control device 7 has a problem and enters into a warning state.

Here, when it is detected that the warning flag is turned ON, the control unit 35 determines that the endoscope balloon control unit 7 is in a warning state because of pressing down of the emergency stop button 23 or the like, and ends the processing routine based on the switch state checking module by the process at the step S20 to keep the current warning process and transfers to the process at the step 7 of Figure 12.

On the other hand, if it is detected that the warning flag is OFF by the determination process at the step S 11, the control unit 35 determines that the emergency stop button 23 is not pressed down and the endoscope balloon control device 7 is not in a warning state, and transfers to the determination process at the step S 12.

At the determination process at the step S12, the control unit 35 determines whether the release button 18a of the remote controller 8 shown in Figure 3 is pressed down or not, and if it determines that it is not pressed down, it transfers to the step S 14. On the other hand, if it determines that it is pressed down, the control unit 35 stops an operation of the first pump 32a corresponding to the release button 18a by the process at the step S13 and controls the duct switching unit 33 and the first amount of flow adjustment valve 32c so as to release the duct, and then it transfers to the step S20 and ends the processing routine based on the first pump switch state checking module.

In the determination process at the step S14, the control unit 35 determines whether the stop button 21a of the remote controller 8 shown in Figure 3 is pressed down or not, and if it determines that it is not pressed down, it transfers to the step S16. On the other hand, if it determines that it is pressed down, the control unit 35 controls the duct switching unit 33 and the first amount of flow adjustment valve 32c so as to close the duct (a duct keeping state) by the process at the step S15, and also stops the operation of the first pump 32a corresponding to the stop button 21 a, and then transfers to the step S20 and ends the processing routine based on the first pump switch state checking module.

In the determination process at the step S16, the control unit 35 determines whether the gas drawing starting button 20a of the remote controller 8 shown in Figure 3 is pressed down or not; and if it determines that it is not pressed down, it transfers to the step S18. On the other hand, if it determines that it is pressed down, the control unit 35 starts the processing routine based on the gas drawing module shown in Figure 15 to be described later in the process at the step S17, performs gas drawing control corresponding to the gas drawing start button 20a, and then transfers to the step S20 and ends the processing routine based on the first pump switch state checking module.

In the determination process at the step S18, the control unit 35 determines whether the gas sending start button 19a of the remote controller 8 shown in Figure 3 is pressed down or not; and if it determines that it is not pressed down, it transfers to the step S20. On the other hand, if it determines that it is pressed down, the control unit 35 starts the processing routine based on a gas sending module shown in Figure 14 to be described later in the process at the step S19, and performs the gas sending control corresponding to the gas sending start button 19a, and then transfers to the step S20 and ends the processing routine based on the first pump switch state checking module.

Next, the processing routine of the gas sending module at the step S19 will be described with reference to Figure 14.

In the embodiment, the control unit 35 is adapted to previously set the pressure maximum value and the pressure upper limit value for previously comparing them with a pressure measured results from the fist and the second sensors 34a and 34b. The pressure upper limit value and the pressure maximum value fulfill relationship of the pressure upper limit value < the pressure maximum value. In such a case, the pressure maximum value means the pressure value equal to cause each of the balloons 9 and 11 to be blown up to a dangerous state, and the pressure upper limit value means a pressure value equal to cause each of the balloons 9 and 11 to be blown up and fixed to the intestines.

When the control unit 35 executes the process at the step S19, it starts the processing routine of the gas sending module shown in Figure 14.

Then, the control unit 35 compares a measured result from the first pressure sensor 34a (for example, a pressure analog value) and the previously set pressure maximum value in the determination process at the step S21; and if it determines that the measured result is smaller than the pressure maximum value, it transfers to the step S22; and if it determines that it is bigger, it transfers to the process at the step S29.

In the process at the step S29, the control unit 35 stops the operation of the first pump 32a and also controls the duct switching unit 33 and the first amount of flow adjustment valve 32c so as to release the duct. Then, the control unit 35 turns on the warning flag in the process at the step S30, then it transfers to the step S37 and ends the processing routine based on the gas sending module.

In the process at the step S22, the control unit 35 compares a measured result of measuring a gas sending time by an inner timer (gas sending time) and a previously set maximum gas sending time; and if it determines that the gas sending time is shorter than the maximum gas sending time, it transfers to the step 23; and if it determines that the gas sending time is longer than the maximum gas sending time, it transfers to the process at the step S31.

In the process at the step S31, the control unit 35 stops the operation of the first pump 32a as in the process at the step S29, and controls the duct switching unit 33 and the first amount of flow adjustment valve 32c so as to release the duct. Then, the control unit 35 turns on the warning flag in the process at the step S32 as in the process at the step S30, then transfers to the step S37 and ends the processing routine based on the gas sending module.

Then, the control unit 35 determines whether the counter value of the timer counter for counting one for every 20 msec of the timer is equal to 1 or not in the determination process at the step S23; and if it determines that it is equal, it stops the operation of the first pump 32a in the process at the step S33 and also controls the duct switching unit 33 and the first amount of flow adjustment valve 32c to keep the duct state. Then, the control unit 35 obtains a measured pressure value by performing pressure measurement by the first and the second pressure sensors 34a and 34b in the process at the step S34 and transfers to the step S24.

In the determination process at the step S23, if it determines that the counter value of the timer counter is not equal to 1, the control unit 35 transfers to the step S24.

Then, the control unit 35 obtains a measured pressure value by performing pressure measurement by the first pressure sensor 34a and compares the obtained measured pressure value (the measured pressure value obtained at the step S34 if it did via a loop of the steps S33 and S34) and a previously set pressure upper limit value in the determination process at the step S24; and if it determines that the measured result is smaller than the pressure upper limit value, it transfers to the step S25; and if it determines that the measured result is bigger than the pressure upper limit value, it transfers to the process at the step S35.

In the process at the step S35, the control unit 35 stops the operation of the first pump 32a and also controls the duct switching unit 33 and the first amount of flow adjustment valve 32c so as to keep the duct state, then it transfers to the step S37 and ends the processing routine based on the gas sending module.

Next, the control unit 35 compares the amount of flow counter value and a predetermined amount of flow of gas sent previously set (amount of flow of gas sent) in the determination process at the step S25; and if it determines that the amount of flow counter value is bigger than the predetermined amount of gas sent, it stops the operation of the first pump 32a in the process at the step S36 and also controls the duct switching unit 33 and the first amount of flow adjustment valve 32c so as to keep the duct state, and then it transfers to the step S37 and ends the processing routine based on the gas sending module.

The amount of flow counter value is a variable for determining how much amount of gas can be sent in 200 msec (20 msec x 10), and can be set from 0 to 9 according to the timer counter value from 0 to 9, for example. Actually, the amount of flow counter (not shown) counts the amount of flow counter value for every 20 msec. The predetermined amount of gas sent indicates an approximate amount of gas sent (amount of gas sent) which can be sent for every 20 msec of the endoscope balloon control device 7.

If the amount of flow counter value is smaller than the predetermined amount of gas sent in the determination process at the step S25, the control unit 35 controls the duct switching unit 33 to set a gas sending duct based on the gas sending start buttons 19a and 19b in the process at the step S27. Then, the control unit 35 adds 1 to the amount of flow counter value in the process at the step S27 and then adds 1 to the gas sending time in the process at the following step S28, and transfers to the step S37 and ends the processing routine based on the gas sending module.

When the processing routine based on the gas sending module ends, the control unit 35 executes the process based on the second pump switch state checking module at the step S7 shown in Figure 12 assuming that the processing routine based on the switch state checking module shown in Figure 13 finished, and thereafter repeatedly executes the processes according to the processing routine shown in Figure 12. Only the operation of the second pump switch state checking module, and a pump, a switch and an object of control are changed, thus, the description of them will be omitted.

Next, the processing routine of the gas drawing module at the step S 17 shown in Figure 13 will be described with reference to Figure 15.

When the control unit 35 executes the process of the step S17, it starts the process routine of a gas drawing module shown in Figure 15.

Then, the control unit 35 compares the measured result of the gas drawing time measured by the inner timer (a gas drawing time) and a previously set maximum gas drawing time in the determination process at the step S40; and if it determines that the gas drawing time is shorter than the maximum gas drawing time, it transfers to the step S41; and if it determines that the gas drawing time is longer than the maximum gas drawing time, it transfers to the process at the step S47.

In the process at the step S41, the control unit 35 stops the operation of the first pump 32a and also controls the duct switching unit 33 and the first amount of flow adjustment valve 32c to release the duct. Then, the control unit 35 turns on the warning flag in the process at the step S48 and then transfers to the step S53 and ends the processing routine based on the gas drawing module.

Then, the control unit 35 determines whether the counter value of the timer counter for counting 1 for each 20 msec of the timer is equal to 1 or not in the determination process at the step S41; and if it determines that it is equal, it stops the operation of the first pump 32a in the process at the step S49 and also controls the duct switching unit 33 and the first amount of flow adjustment valve 32c to keep the duct state. Then, the control unit 35 obtains a measured pressure value by performing pressure measurement by the first pressure sensor 34a in the process at the step S50, and transfers to the step S42.

In the determination process at the step S41, if it determines that the counter value of the timer counter is not equal to 1, the control unit 35 transfers to the step S42.

Then, the control unit 35 obtains a measured pressure value by performing pressure measurement by the first pressure sensor 34a and compares the obtained measured pressure value (the measured pressure value obtained at the step S50 if it did via a loop of the steps S49 and S50) and a previously set pressure lower limit value in the determination process at the step S42; and if it determines that the measured result is smaller than the pressure lower limit value, it transfers to the step S51; and if it determines that the measured result is bigger than the pressure lower limit value, it transfers to the process at the step S43.

In the process at the step S51, the control unit 35 stops the gas drawing operation of the first and the second pumps 32a and 32b and also controls the duct switching unit 33 and the first and the second amount of flow adjustment valves 32c and 32d so as to release the duct, then it transfers to the step S53 and ends the processing routine based on the gas drawing module.

Next, the control unit 35 compares the amount of flow counter value and a predetermined amount of gas drawn previously set (amount of gas drawn) in the determination process at the step S43; and if it determines that the amount of flow counter value is bigger than the predetermined amount of gas drawn, it stops the gas drawing operation of the first pump 32a in the process at the step S52 and also controls the duct switching unit 33 and the first amount of flow adjustment valve 32c so as to keep the duct state, and then it transfers to the step S54 and ends the processing routine based on the gas drawing module. The predetermined amount of gas drawn indicates an approximate amount of gas drawn (amount of flow of air drawn) which can be drawn for every 20 msec of the endoscope balloon control unit 7.

If it determines that the amount of flow counter value is smaller than the predetermined amount of gas drawn in the determination process at the step S43, the control unit 35 controls the duct switching unit 33 to set a gas drawing duct based on the gas drawing start button 20a in the process at the step S44. Then the control unit 35 adds 1 to the amount of flow counter value in the process at the step S45 and adds 1 to the gas drawing time in the process at the following step S46, and transfers to the step S53, and ends the processing routine based on the gas drawing module.

When the processing routine based on the gas drawing module ends, the control unit 35 executes the process based on the first pump control module at the step S7 shown in Figure 12 assuming that the processing routine based on the switch state checking module shown in Figure 13 finished, and thereafter repeatedly executes the process according to the processing routine shown in Figure 12.

As mentioned above, according to the embodiment, as the endoscope balloon control device 7 can adjust the amount of gas sent to and the amount of gas drawn from each of the balloons 9 and 11, it can be adapted to various materials of balloon or various parts. The endoscope balloon control device 7 can prevent a serial gas sending operation or a serial gas drawing operation which occurs when a duct such as the first and the second gas sending tubes 13 and 14 and the like, for example, comes off.

The endoscope balloon control device 7 can control the pressure inside the balloon to release the duct by detecting that the maximum gas sending time, the maximum gas drawing time, the maximum gas sending pressure and the maximum gas drawing pressure are exceeded so that it allows the procedure to be performed without needing much strength to be added to the intestine wall.

In the embodiment, although a configuration of the remote controller 8 connected to the endoscope balloon control device 7 has been described, the present invention is not limited to that and can be configured with an operation unit 2A of the endoscope 2, which is at hand of the operator, or a foot switch for controlling the endoscope balloon control device 7 at the foot of the operator, for example.

The controller 8 may be adapted to send various remote control operation signals using infrared radiation, receive the infrared radiation by a photoreceptor provided for the endoscope balloon control device 7 and capture the remote control signal. That further facilitates an operator's manipulation.

### (Embodiment 2)

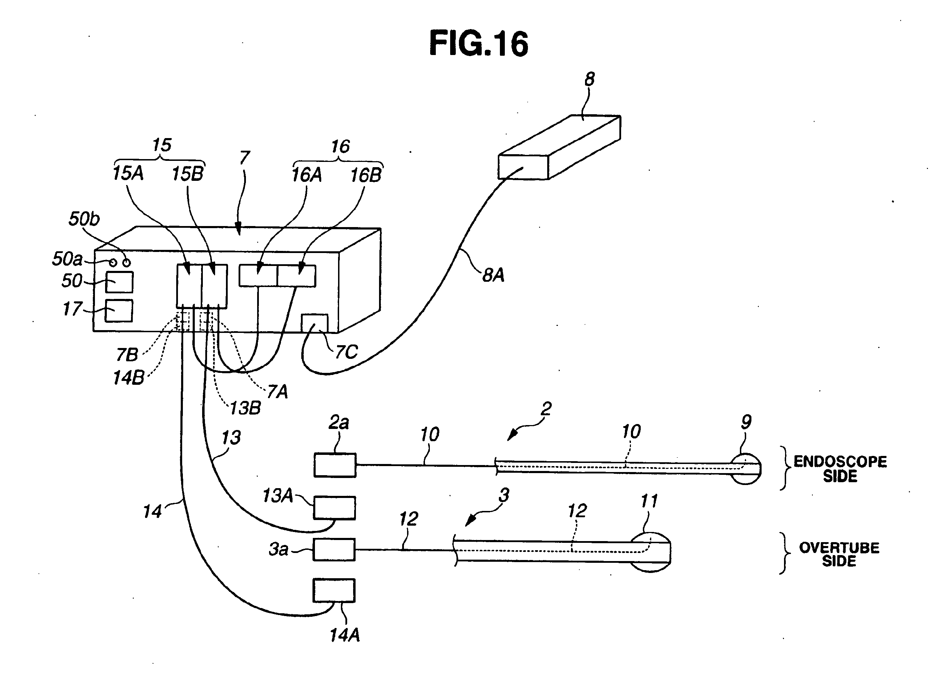

Next, an endoscope balloon control device according to the second embodiment will be described with reference to Figure 16 to Figure 22.

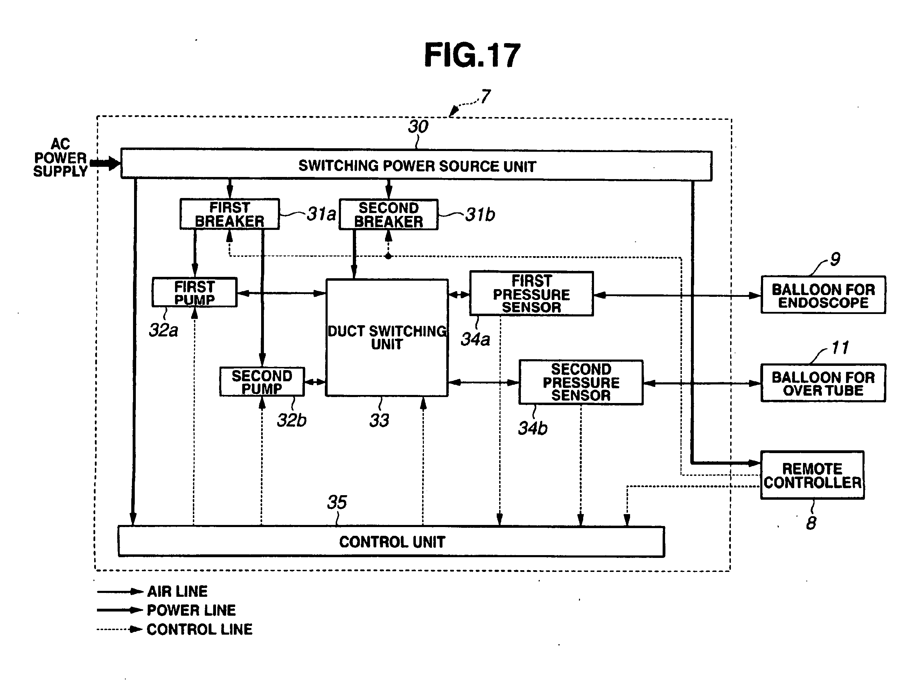

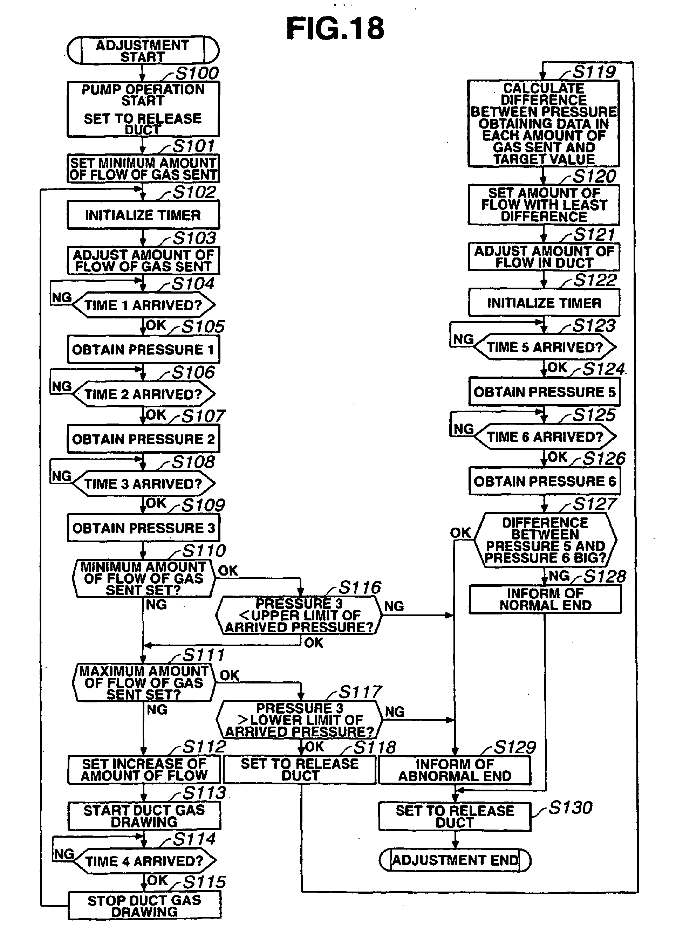

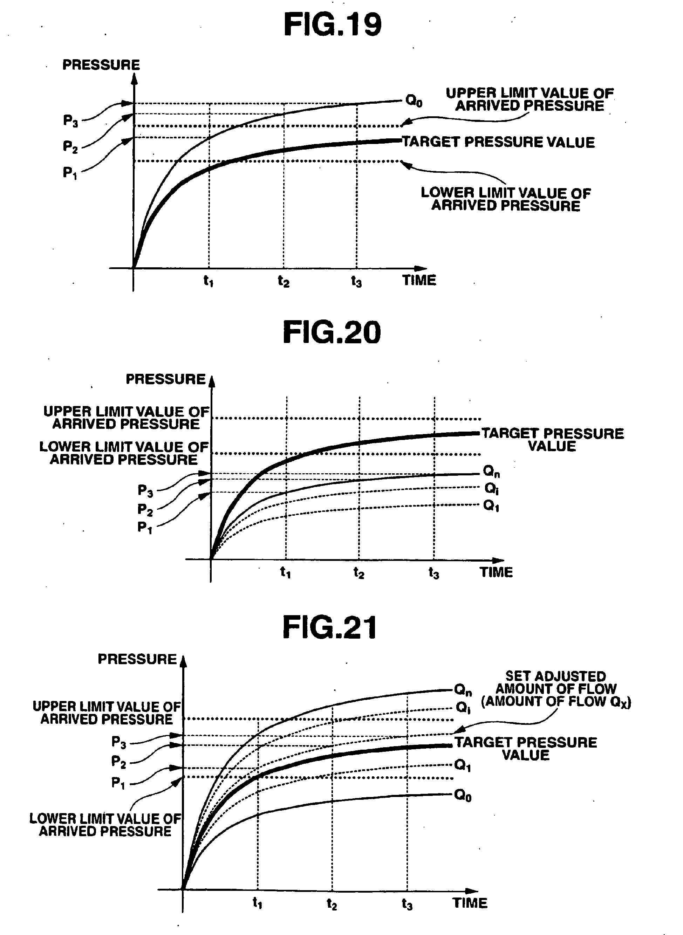

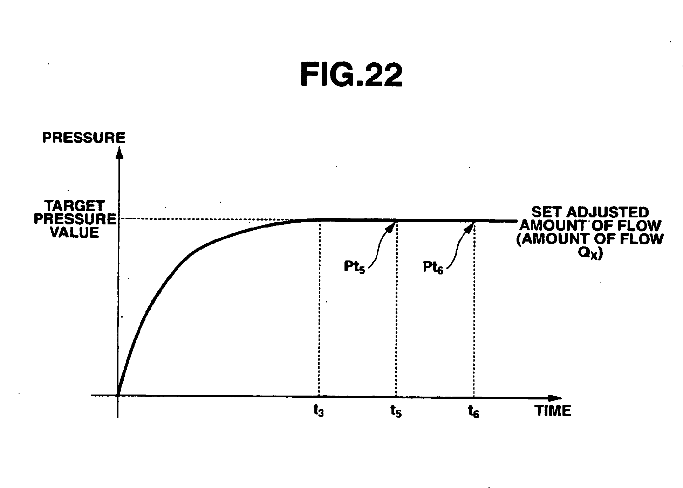

From Figure 16 to Figure 22 relate to the embodiment 2; Figure 16 is a configuration diagram showing an outlined configuration of an endoscope balloon control device of the second embodiment; Figure 17 is a block diagram showing an inner configuration of the endoscope balloon control device of Figure 16; Figure 18 is a flowchart showing control contents of an endoscope balloon control device according to the second embodiment; Figure 19 is a graph showing relationship between an inner pressure of a duct and a gas sending time when it is determined as abnormal adjustment ending information in the minimum amount of flow setting of a pump in a flowchart of Figure 18; Figure 20 is a graph showing relationship between an inner pressure of a duct and a gas sending time when it is determined as abnormal adjustment ending information in the maximum amount of flow setting of a pump in a flowchart of Figure 18; Figure 21 is a graph showing relationship between an inner pressure of a duct and a gas sending time when no abnormal state is recognized even if the amount of flow setting of the pump is changed from the minimum amount of flow setting to the maximum amount of flow setting in a flowchart of Figure 18; and Figure 22 is a graph showing relationship between an inner pressure of a duct and a gas sending time when change in an inner pressure of a duct is to be checked by making an inner pressure of a duct a predetermined inner pressure value to be an approximate equilibrium state after the amount of flow setting of the pump is made an amount of flow set after adjustment in the flowchart of Figure 18.

The entire configuration of an endoscope system 1 of the embodiment is almost the same as that of the endoscope system 1 shown in Figure 1. The endoscope balloon control device 7 used in the endoscope system 1 is provided with an examination switch 50 and the first LED (Light Emitting Diode) 50a, which is an abnormal state information unit, and the second LED 50b, which is also an abnormal state information unit, on an external surface for the configuration of the embodiment 1 as shown in Figure 16.

In the embodiment, a display device 16A of a pressure display device 16 performs display of an inner pressure value of the balloon 9 measured based on an inner pressure of a duct in which the first pressure sensor 34a, which is a pressure measuring unit, is communicating with the balloon 9. The display device 16B of the pressure display device 16 performs display of an inner pressure value of the balloon 11 measured based on an inner pressure of a duct in which the second pressure sensor 34b, which is a pressure measuring unit, is communicating with the balloon 11.

The examination switch 50 is a switch which can perform a predetermined examination on the balloon 9 and the balloon 11, which are predetermined ducts communicating with an endoscope balloon control device 7 as it is turned on. Detailed contents of the predetermined examination will be described later.

When the predetermined examination ends, in response to the contents of the determination result of the control unit 35 provided inside the endoscope balloon control device 7, the examination result of the balloon 9 is displayed on the first LED 50a and the examination result of the balloon 11 is displayed on the second LED 50b.

The first LED 50a and the second LED 50b, which are abnormal state informing units, can inform an operator of abnormality of the balloon 9 or the balloon 11 by means of lighting on if the examination normally ends, and flashing if the examination abnormally ends to inform of abnormality.

The first LED 50a and the second LED 50b, which are an abnormal state information units, may not be that mentioned above as an informing unit when a predetermined examination ends, and may be adapted to facilitate recognition of the abnormal ending of the examination by the balloon 9 or the balloon 11 by using different colors of LEDs for normal ending and abnormal ending such as lighting a blue LED on when a predetermined examination ends normally and lighting a red LED on when a predetermined examination ends abnormally.

The abnormal state informing unit may not only be a visual informing unit by the LED. For example, an informing unit using sound so as to draw attention of an operator by giving out a predetermined sound at the same time of lighting of an LED, when contents of an examination result meaning that the balloon 9 or the balloon 11 is abnormal is received.

Further, in Figure 16, although the number of LEDs provided on an external surface of the endoscope balloon control device 7 is two, the number of LEDs is not limited to two and it may be increased as required.

Next, by using Figure 17, a structure, control contents and operations for the control contents of the endoscope balloon control device 7 will be described.

As shown in Figure 17, although the endoscope balloon control device 7 has almost the same configuration as that of the embodiment 1, the first and the second amount of flow adjustment valves 32c and 32d and the third breaker 31c are deleted and the first and the second pressure sensors 34a and 34b are provided between the duct switching unit 33 and the balloon 9 of the endoscope 2 and the balloon 11 of the overtube 3. In some cases, it may have the same configuration as that of the embodiment 1 (see Figure 4) without deleting the first and the second amount of flow adjustment valves 32c and 32d and the third breaker 31c.

When the power source switch 17 or the power source button 22 provided for the remote controller 8 is turned on, the switching power source unit 30 supplies power needed for each of the first pump 32a, the second pump 32b, the duct switching unit 33, the remote controller 8 and the control unit 35 based on power supplied from alternators (not shown) provided for the outside of endoscope balloon control device 7.

When power is supplied to each unit, the first pump 32a and the second pump 32b start operating as an initial setting state, the duct switching unit 33 is in a releasing state, i.e., in a state where the inner pressure of a duct (not shown) provided in the duct switching unit 33 is almost the same as the outside air pressure.

If he first pump 32a is communicated with a duct (not shown) provided in the duct switching unit 33 and the balloon 9 is attached to the endoscope balloon control device 7, it is communicated with the first pump 32a, which is a predetermined duct, based on contents of a control signal sent from the control unit 35, and sends or draws gas to or from the first duct to the balloon 9.

If the second pump 32b is communicated with a duct (not shown) provided in the duct switching unit 33 and the balloon 11 is attached to the endoscope balloon control device 7, it is communicated with the second pump 32b, which is a predetermined duct, based on contents of a control signal sent from the control unit 35, and sends or draws gas to or from the second duct to the balloon 11.

The first pressure sensor 34a is a pressure measuring unit and sends measured inner pressure value of the balloon 9 to the control unit 35. The second pressure sensor 34b is a pressure measuring unit and sends measured inner pressure value of the balloon 11 to the control unit 35.

The control unit 35 is control means provided with an abnormality determining section in a predetermined examination to be described later, and controls the first pump 32a, the second pump 32b and the duct switching unit 33 based on an inner pressure value of the balloon 9 measured by the first pressure sensor 34a and an inner pressure value of the balloon 11 measured by the second pressure sensor 34b. The control unit 35 sends a signal for performing enlargement and reduction of the balloon 9 and the balloon 11 to the first pump 32a, the second pump 32b and the duct switching unit 33 based on a signal in response to manipulation of various buttons provided on the remote controller 8.

Accordingly, the first pump 32a, the second pump 32b and the duct switching unit 33 are controlled so that the balloon 9 and the balloon 11 can perform enlargement and reduction in conjunction with manipulation of various buttons (see Figure 3) provided on the remote controller 8.

The control unit 35 is adapted to have an abnormality determining section (not shown) comprising a central processing unit (CPU), a memory and the like in its inside for previously determining a predetermined time, a predetermined setting value and a predetermined contents of control in a method such as programming so as to automatically perform predetermined control by the predetermined time and the predetermined setting value in conjunction with a timer (not shown) (a timer counter).

The other configuration is the same as that of the embodiment 1 (see Figure 4).

Next, with the abovementioned configuration, a predetermined examination which can be performed by using the endoscope balloon control device 7, i.e., an abnormality examination, which is a method for determining abnormality on the balloon 9 and the balloon 11, which are predetermined ducts, will be described with reference to Figure 18.

When an abnormality examination is performed on the balloon 9 and the balloon 11 by using the endoscope balloon control device 7, first, a connector 13A is attached to a connector 2a and an air supplying tube 10 and the first gas sending tube (an endoscope balloon gas sending tube) 13 are communicated with each other and a connector 14A is attached to a connector 3a and an air supplying tube 12 and the second gas sending tube (a balloon gas sending tube for an overtube) 14 are communicated with each other. As such, after making it possible to send gas to the balloon 9 and the balloon 11, the power source switch 17 or the power source button 22 provided for the remote controller 8 is turned on.

If the examination switch 50 is turned on in the state, an abnormality examination on the balloon 9 and the balloon 11 starts. The contents of the abnormality examination, which is the abovementioned predetermined examination, and a procedure and a predetermined setting value in the abnormality examination are previously stored and set as a program, data or the like in a memory and the like (not shown) in the control unit 35.

After starting the examination, the control unit 35 operates the first pump 32a and the second pump 32b and also sets a duct release state in the duct switching unit 33 (step S100). Then, the control unit 35 sets to send a control signal for sending gas of air by the minimum amount of gas sent (Q0) to the first pump 32a, the second pump 32b and the duct switching unit 33 (step S101).

Then, after a timer (not shown) is initialized (step S102) as the first step, i.e., as the minimum amount of gas sent adjustment step (the step S102 and the step S103), the control unit 35 sends the control signal, and the duct switching unit 33 having received the control signal adjusts the amount of gas sent so as to send gas of air by the minimum amount of gas sent (Q0) to the balloon 9 and the balloon 11 from the first pump 32a and the second pump 32b by using the amount of flow adjustment unit such as a valve or the like (not shown) included in the duct switching unit 33.

In the embodiment, although it is described like below by assuming that the minimum amount of gas sent (Q0) to the balloon 9 and the balloon 11 is the same amount of gas sent, the minimum amount of gas sent (Q0) may be different amount of gas sent respectively.

After the amount of gas sent adjustment step is performed, an inner pressure obtaining step (from the step S 104 to the step S109) for the control unit 35 to obtain an inner pressure value at a time when a predetermined time passed is performed continuously.

At the inner pressure value obtaining step, first, gas is sent to the balloon 9 and the balloon 11 based on the minimum amount of gas sent (Q0), and when the time 1 (t1), which is a predetermined time previously set in the control unit 35, passes (the step S104), the control unit 35 obtains a pressure 1 (P1), which is an inner pressure value of the balloon 9 measured by the first pressure sensor 34a and an inner pressure value of the balloon 11 measured by the second pressure sensor 34b (the step S105).

Then, when the time 2 (t2), which is a predetermined time previously set in the control unit 35, passed (the step S106), the control unit 35 obtains a pressure 2 (P2), which is an inner pressure value of the balloon 9 measured by the first pressure sensor 34a and an inner pressure value of the balloon 11 measured by the second pressure sensor 34b (the step S107).

Further, when the time 3 (t3), which is a predetermined time previously set in the control unit 35, passed (the step S108), the control unit 35 obtains a pressure 3 (P3), which is an inner pressure value of the balloon 9 measured by the first pressure sensor 34a and an inner pressure value of the balloon 11 measured by the second pressure sensor 34b (the step S 109).

After obtaining the pressure 3 (P3) at the time 3 (t3), the control unit 35 compares the upper limit value of the arrived pressure which is a first threshold previously set in the control unit 35 and the pressure 3 (P3) by using an abnormality determining section (not shown) included its inside (the step S110 and the step S116). At this moment, as shown in Figure 19, if the pressure 3 exceeds the upper limit value of the arrived pressure, the control unit 35 determines that any of the first pump 32a, the second pump 32b and the duct is abnormal as the second step.

The determination result is visually informed by the first LED 50a or the second LED 50b, which is an abnormal state informing unit, as the third step (the step S129), so that an operator, an assistant of the operator or the like can recognize that any of the first pump 32a, the second pump 32b and the duct is abnormal from the information. Then, after the abnormal state is informed, the control unit 35 ends the examination by setting a duct release state at the duct switching unit 33 (the step S130).

If the pressure 3 (P3) does not reach the upper limit of arrived pressure in the abovementioned comparison between the upper limit of the arrived pressure and the pressure 3 (P3), determination is performed on whether the current amount of flow of gas sent is the maximum amount of flow of gas sent (Qn) or not (the step S111). As the amount of flow of gas sent is set as the minimum amount of flow of gas sent (Q0), the control unit 35 sets to send a control signal for performing sending gas of air by increasing the amount of flow of gas sent from the minimum amount of flow of gas sent (Q0) to a predetermined setting value (Q1) (the step S112) as an amount of flow of gas sent setting step (from the step S112 to the step S115). After performing the setting, the control unit 35 sends a control signal for making a state of the duct to a gas drawing state to the first pump 32a, the second pump 32b and the duct switching unit 33.

When the first pump 32a, the second pump 32b and the duct switching unit 33 start gas drawing of the duct (the step S113), and the balloon 9 and the balloon 11 start reduction. Then, when a time 4 (t4) at which both of the inner pressure values of the balloon 9 and the balloon 11 become almost 0 passes (the step S114), the control unit 35 stops gas drawing of the duct to the first pump 32a, the second pump 32b and the duct switching unit 33 (the step S115).

Then, after the timer (not shown) is initialized again (the step S102), the control unit 35 sends a control signal to make it an amount of gas sent of a predetermined setting value (Q1) to the first pump 32a, the second pump 32b and the duct switching unit 33.

The control unit 35 repeats the processes from the step S3 to the step S16 until the setting value of the amount of flow of gas sent Qi (i=0, 1, ..., n-1, n) increases step by step to the amount of flow of gas sent of the maximum amount of flow of gas sent (Qn).

That is to say, an amount of flow of gas sent adjustment step in which a timer (not shown) is initialized and adjustment of each of the amounts of flow of gas sent to the duct (Q1, Q2, ..., Qi, Qn-1) is performed by the first pump 32a, the second pump 32b and the duct switching unit 33 based on a control signal sent from the control unit 35 (the step S3 and the step S4); an inner pressure value obtaining step in which the control unit 35 obtains the pressure 1 (P1) when the time 1 (t1) passed, the pressure 2 (P2) when the time 2 (t2) passed, and the pressure 3 (P3) when the time 3 (t3) passed in each of the amount of flow of gas sent (Q1, Q2, ..., Qi, ..., Qn-1) (from the step S 104 to the step S109); and an amount of flow of gas sent setting step in which after gas drawing of the duct starts and the time 4 (t4) passed, gas drawing of the duct stops and each of the gas sending amounts (Q2, Q3, ..., Qi+1, ..., Qn) is set to the duct in the next measurement (from the step S 112 to the step S 115) are repeated by the predetermined number of times previously set in the control unit 35 until it reaches the maximum amount of flow of gas sent (Qn).

In the embodiment, the predetermined number of times is assumed to be (n+1) from the minimum amount of flow of gas sent (Q0) to the maximum amount of flow of gas sent (Qn).

Then, after the (n+1) times of the amount of flow of gas sent setting step is performed, the control unit 35 sends a control signal, and the duct switching unit 33 having received the control signal adjusts the amount of flow of gas sent so that the duct switching unit 33 sends gas to the balloon 9 and the balloon 11 by the maximum amount of flow of gas sent (Qn) in conjunction with the first pump 32a and the second pump 32b by using an amount of flow adjustment unit such as a valve and the like (not shown) included its inside as the fourth step, i.e., the maximum amount of flow of gas sent adjustment step (the step S102 and the step S103).

After the inner pressure value obtaining step (from the step S 104 to the step S109) ends by the maximum amount of flow of gas sent (Qn), the control unit 35 compares the lower limit of the arrived pressure, which is a second threshold previously set in the control unit 35, and a pressure 3 (P3) by using an abnormality determining section (not shown) included its inside (the step S111 and the step S117).

At this moment, if the pressure 3 (P3) does not reach the lower limit of the arrived pressure as shown in Figure 20, the control unit 35 determines that any of the first pump 32a, the second pump 32b and the duct is abnormal as a fifth step.

The determination result is visually informed of by the first LED 50a or the second LED 50b, which is an abnormal state informing unit, as a sixth step (the step S129), and an operator or an assistant or the like of the operator can recognize that the first pump 32a or the second pump 32b is abnormal. After an abnormal state is informed, the control unit 35 ends an examination by setting a duct release state by the duct switching unit 33 (the step S130).

If a pressure 3 (P3) exceeds the lower limit of the arrived pressure in the comparison between the lower limit of the arrived pressure and the pressure 3 (P3) (the step S 117), the control unit 35 sets a duct release state (the step S118), and then performs a calculation for obtaining a value of a difference between each pressure 3 (P3) obtained by each of the amount of flow of gas sent and a target pressure value, which is a predetermined pressure value previously set in the control unit 35, on the pressure 3 (P3) by (n+1) times from the minimum amount of flow of gas sent (Q0) to the maximum amount of flow of gas sent (Qn), respectively (the step S 119).

Further, the control unit 35 compares the difference values among the (n+1) times and sends a control signal to the first pump 32a, the second pump 32b and the duct switching unit 33 so that it can send gas to the balloon 9 and the balloon 11 by the amount of flow of gas sent for which the pressure 3 (P3) with the least difference is measured, i.e., by the amount of flow set after adjustment (Qx) as shown in Figure 21 (the step S 120 and the step S121).

At this moment, a target pressure value corresponding to each of the time 1 (t1), the time 2 (t2) and the time 3 (t3) may be previously set in the control unit 35, and the control unit 35 may calculate a square of respective differences for the pressure 1 (P1), the pressure 2 (P2), and the pressure 3 (P3) for (n+1) number of times from the minimum amount of flow of gas sent (Q0) to the maximum amount of flow of gas sent (Qn) based on the target pressure value, and make the amount of flow of gas sent for which the calculated value obtained by the calculation becomes the least the amount of flow set after adjustment (Qx).

The amount of flow set after adjustment (Qx) set in the abovementioned manner is an amount of flow of gas sent set to the balloon 9 and the balloon 11 respectively to be actually used by an operator. Therefore, even when an operator performs treatment as the operator uses the balloon 9 and the balloon 11 with different materials and capacities according to the purpose, air can be sent by an optimal amount of flow of gas sent to respective of the balloon 9 and the balloon 11, i.e., the amount of flow set after adjustment (Qx).

As an abnormal examination on the balloon 9 and the balloon 11 in the amount of flow set after adjustment (Qx) after the step S 121, first, a timer (not shown) is initialized (the step S121) and air is sent to the balloon 9 and the balloon 11.

When the time 3 (t3) passed, the control unit 35 sets a duct keeping state, and then the control unit 35 obtains a pressure 5 (Pt5), which is an inner pressure value of the balloon 9 measured by the first pressure sensor 34a and an inner pressure value of the balloon 11 measured by the second pressure sensor 34b, i.e., the first inner pressure value, at the time 5 (t5), which is a first time previously set in the control unit 35 and also a time after the time 3 (t3) (the step S123 and the step S124).

Further, the control unit 35 obtains a pressure 6 (Pt6), which is an inner pressure value of the balloon 9 measured by the first pressure sensor 34a and an inner pressure value of the balloon 11 measured by the second pressure sensor 34b, i.e., the second inner pressure value, at a time 6 (t6), which is a second time previously set in the control unit 35 and also a time after a time 5 (t5) (the step S125 and the step S126).

After obtaining the pressure 6 (Pt6) at the time 6 (t6), the control unit 35 calculates for obtaining an absolute value of a difference between the pressure 5 (Pt5) and the pressure 6 (Pt6). Further, the control unit 35 compares between a predetermined threshold previously set in the control unit 35 and the absolute value by using control means included its inside (the step S127); and if the absolute value exceeds the predetermined threshold, it determines that any of the balloon 9, the balloon 11 or the duct is abnormal.

The determined result is visually informed by the first LED 50a or the second LED 50b, which are abnormal state information units (the step S129) so that an operator or an assistant or the like of the operator can recognize that the balloon 9 or the balloon 11 is abnormal. After an abnormal state is informed of, the control unit 35 sets a duct release state in the duct switching unit 33 to end the examination by using control means included its inside (the step S130).

In the embodiment, although it is assumed that a target pressure value of the first pump 32a and the second pump 32b, the amount of flow set after adjustment (Qx) and the measured value of each duct are the same respectively, the flow may be performed individually on each of the pumps and ducts and an individual amount of flow set after adjustment (Qx) may be obtained for an individual target pressure value through individual measurement.

If the absolute value of a difference between the predetermined threshold and the pressure 5 (Pt5) and the pressure 6 (Pt6) does not reach the predetermined threshold, the control unit 35 determines that all of the balloon 9 and the balloon 11 and the duct are normal. The determined result is visually informed by the first LED 50a or the second LED 50b, which is an abnormal state information unit (the step S128), and an operator or an assistant or the like of the operator can recognize that the balloon 9 and the balloon 11 are normal. After the normal state is informed of, the control unit 35 sets a duct release state in the duct switching unit 33 to end the examination by using control means included its inside (the step S 130).

In such a configuration, the endoscope balloon control unit 7 of the embodiment has a function of performing an abnormality examination as mentioned above on the balloon 9 and the balloon 11 so that an operator or an assistant or the like of the operator can simply and automatically recognize abnormality such as poor air sealing or the like in the balloon 9 and the balloon 11 at a site of performing treatment on a living body. As such, the possibility that blowout or poor fixing of the balloon 9 or the balloon 11 occurs in a living body which may be caused by abnormality of the balloon 9 or the balloon 11 itself can be eliminated.

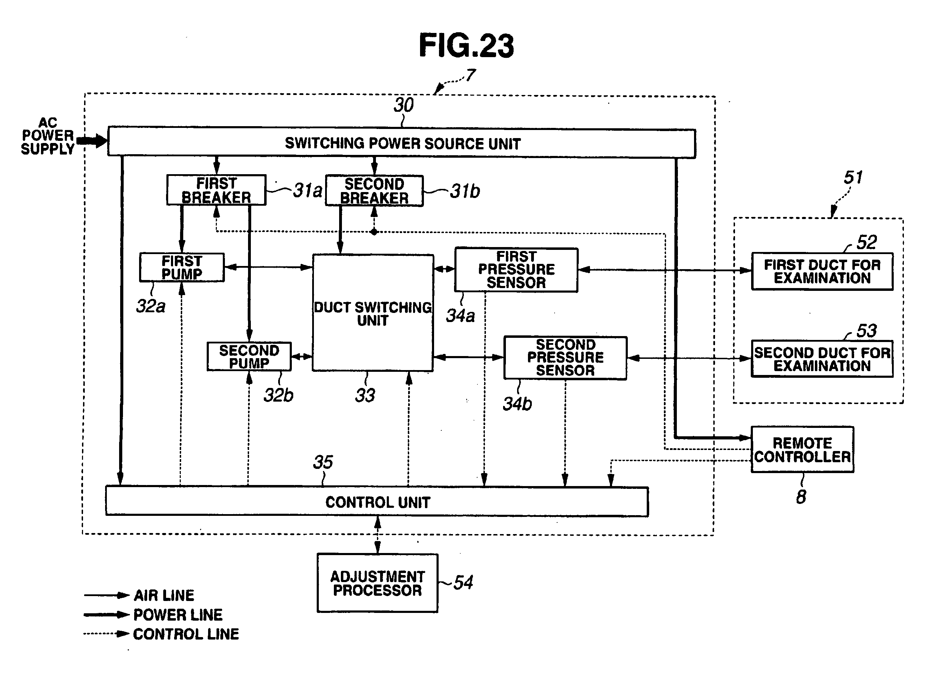

The endoscope balloon control device 7 can perform an abnormality examination by attaching an examination jig 51 and an adjustment processor 54 which substitute for the balloon 9 and the balloon 11 as shown in Figure 23. The examination jig 51 has a first examination duct 52 and a second examination duct 53 which are made of a predetermined material and have predetermined capacities, for example.

The adjustment processor 54 is a terminal device such as a personal computer, for example, and can communicate with the control unit 35 by using wired or wireless communication means and can change a predetermined time, a predetermined value, predetermined contents of control previously set in the control unit 35 to any setting values by using the communication means.

For example, in the abovementioned abnormal examination, although the number of times that the control unit 35 obtains an inner pressure value in the inner pressure value obtaining step (from the step S104 to the step S109) is three times of the time 1 (t1), the time 2 (t2) and the time 3 (t3), the number of time may be set to any number of times by using the adjustment processor 54.

Further, by connecting a display unit such as a monitor or the like (not shown) to the adjustment processor 54, an abnormal informing unit can be provided for visually displaying normal ending or abnormal ending of an examination on the display unit as well as lighting, flashing and the like of the first LED 50a and the second LED 50b provided for the external surface of the endoscope balloon control device 7.

If the abovementioned abnormality examination is performed in such a configuration, an abnormality examination as an abnormality determining method of the first pump 32a, the second pump 32b and the duct switching unit 33 can be performed by performing an examination from the first step to the sixth step.

By performing setting of the amount of flow set after adjustment (Qx) on a predetermined examination jig 51, the amount of flow of gas sent of air of a plurality of endoscope balloon control device 7, which use the balloon 9 and the balloon 11 corresponding to the first examination duct 52 and the second examination duct 53 and are the same model, can be adjusted to a predetermined amount of flow set.

With the abovementioned operation, an examiner can recognize abnormality of an amount of flow adjustment unit provided inside the endoscope balloon control device 7, i.e., the pump 32a, the second pump 32b and the duct switching unit 33 in an examination and the like at a manufacturing time of the endoscope balloon control devices 7 which are the same model. Where the amount of flow adjustment unit is normal, the examiner can simply and automatically adjust the amount of flow of gas sent of air of a plurality of endoscope balloon control devices 7 which are the same model to the amount of flow set after adjustment (Qx) which is approximately the same amount of flow of gas sent.

Therefore, according to the embodiment, in addition to obtaining the same effects as those of the embodiment 1, an endoscope balloon control device and a method for determining abnormality of the endoscope balloon control device which can simply and automatically determine abnormality in a balloon duct in controlling the amount of flow of gas sent or gas drawn to or from the balloon can be realized.

In the present invention, it is apparent that different embodiments in a wide range can be adapted based on the present invention without departing from the scope of the present invention. The present invention is not limited by particular embodiments except for being limited by the range of the attached claims.

## Claims

1. An endoscope balloon control device (7) comprising a first pump (32a) for supplying or discharging gas to or from a first balloon (9) for fixing the endoscope, said balloon being attached to a peripheral part of a tip part of an inserting unit (2B) of an endoscope (2); and a control unit (35) for controlling a pressure inside the first balloon(9) by operating the first pump (32a) based on the measured result by measuring a pressure of gas inside the first balloon(9), **characterized in that**
the control unit (35) has an amount of flow detecting unit for detecting the amount of flow of gas sent or drawn to or from the first balloon (9), and controls the amount of flow of gas sent or drawn to or from the first balloon(9) by operating the first pump (32a) based on the detected result by the amount of flow detecting unit.

2. The endoscope balloon control device according to claim 1, **characterized by** comprising a second pump (32b) for supplying or discharging gas to or from a second balloon (11) for fixing the endoscope, said balloon being attached to a peripheral part of a tip part of an overtube (3) for the endoscope (2) being inserted therethrough; the amount of flow detecting unit being adapted to detect the amount of flow of gas sent or drawn to or from the first balloon (9) and the second balloon of the overtube (3); wherein the control unit (35) controls the amount of flow of gas sent or drawn to or from each of the first and second balloons (9, 11) by operating the second pump (32b) based on the detected result by the amount of flow detecting unit.

3. The endoscope balloon control device according to claim 1, **characterized in that** the control unit (35) stops an operation of the first pump (32a) when the detected result from the amount of flow detecting unit exceeds a predetermined threshold.

4. The endoscope balloon control device according to claim 2, **characterized in that** the control unit (35) stops an operation of the second pump (32b) when the detected result from the amount of flow detecting unit exceeds a predetermined threshold.

5. The endoscope balloon control device according to claim 1, **characterized by** comprising a remote controller (8) including a plurality of manipulation buttons (18a, 19a, 20a, 21a) and an emergency stop button (23) forgiving indications to the control unit (35); wherein the control unit (35) stops an operation of the first pump (32a) when the emergency stop button (23) of the remote controller (8) is pressed down.

6. The endoscope balloon control device according to claim 2, **characterized by** comprising a remote controller (8) including a plurality of manipulation buttons (18b, 19b, 20b, 21b) and an emergency stop button (23) for giving indications to the control unit (35); wherein the control unit (35) stops an operation of the second pump (32b) when the emergency stop button (23) of the remote controller (8) is pressed down.

7. The endoscope balloon control device according to claim 1, wherein the first pump (32a) is for supplying or discharging gas to or from a duct connected to the first balloon (9) and is further comprising a first pressure measuring unit (34a) for measuring an inner pressure of the first balloon(9) or the duct; a first amount of flow adjustment unit (32c) for adjusting the amount of flow of gas of the first pump (32a) sent to the duct; a first time detecting unit for detecting a gas sending time or a gas drawing time to or from the first balloon(9); wherein the control unit (35) is for adjusting the pressure inside the first balloon (9) by operating the first pump (32a) and controlling the amount of flow of gas sent or drawn to or from the first balloon (9) based on a measured result by the first pressure measuring unit (34a) and a detected result by the amount of flow detecting unit and a detected result by the first time detecting unit; wherein the control unit (35) comprises an abnormality determining section for changing the amount in flow of gas sent from the first pump (32a) to the duct by controlling the first amount of flow adjustment unit, supplying gas to the duct and obtaining a first inner pressure value and a second inner pressure value of the duct measured by the first pressure measuring unit (34a) at a previously set first time and a previously set second time and, if a difference between the first inner pressure value and the second inner pressure value exceeds a previously set threshold, determining that the first balloon (9) is abnormal; and an abnormal state informing unit for informing abnormality of the duct based on the determined result of the abnormality determining section.

8. The endoscope balloon control device according to claim 2, wherein the second pump (32b) is for supplying or discharging gas to or from a duct connected to the second balloon (11) and is further comprising a second pressure measuring unit (34b) for measuring an inner pressure of the second balloon (11) or the duct; a second amount of flow adjustment unit (32d) for adjusting the amount of flow of gas of the second pump (32b) sent to the duct; a second time detecting unit for detecting a gas sending time or a gas drawing time to or from the second balloon (11); wherein the control unit (35) is for adjusting a pressure inside the second balloon (11) by operating the second pump (32b) and controlling the amount of flow of gas sent or drawn to or from the second balloon (11) based on a measured result by the second pressure measuring unit (34b) and a detected result by the amount of flow detecting unit and a detected result by the second time detecting unit; wherein the control unit (35) comprises an abnormality determining section for changing the amount in flow of gas sent from the second pump (32b) to the duct by controlling the second amount of flow adjustment unit, supplying gas to the duct and obtaining a first inner pressure value and a second inner pressure value of the duct measured by the second pressure measuring unit (34b) at a previously set first time and a previously set second time and, if a difference between the first inner pressure value and the second inner pressure value exceeds a previously set threshold, determining that the second balloon (11) is abnormal; and an abnormal state informing unit for informing abnormality of the duct based on the determined result of the abnormality determining section.

9. The endoscope balloon control device according to claim 7, **characterized in that** the abnormality determining section changes the amount of flow of gas of the first pump (32a) sent to the duct to the minimum amount of flow of gas sent or the maximum amount of flow of gas sent by controlling the first amount of flow adjustment unit and supplies gas to the duct and obtains an inner pressure value of the duct measured by the first pressure measuring unit (34a) at a previously set setting time, and if the inner pressure value of the duct exceeds a previously set first threshold when the setting time passed after gas is supplied to the duct by the minimum amount of flow of gas sent by the first pump (32a), or if the inner pressure value of the duct does not reach a previously set second threshold when the setting time passed after gas is supplied to the duct by the maximum amount of flow of gas sent by the first pump (32a), determines that the first pump (32a) is abnormal.

10. The endoscope balloon control device according to claim 8, **characterized in that** the abnormality determining section changes the amount of flow of gas of the second pump (32b) sent to the duct to the minimum amount of flow of gas sent or the maximum amount of flow of gas sent by controlling the second amount of flow adjustment unit and supplies gas to the duct and obtains an inner pressure value of the duct measured by the second pressure measuring unit (34b) at a previously set setting time, and if the inner pressure value of the duct exceeds a previously set first threshold when the setting time passed after gas is supplied to the duct by the minimum amount of flow of gas sent by the second pump (32b), or if the inner pressure value of the duct does not reach a previously set second threshold when the setting time passed after gas is supplied to the duct by the maximum amount of flow of gas sent by the second pump (32b), determines that the second pump (32b) is abnormal.

11. The endoscope balloon control device according to claim 9, **characterized in that** the abnormality determining section performs an amount of flow adjustment to make a difference between an inner pressure value of the duct measured by the first pressure measuring unit (34a) at the setting time and a previously set target value of an inner pressure to the minimum.

12. The endoscope balloon control device according to claim 10, **characterized in that** the abnormality determining section performs an amount of flow adjustment to make a difference between an inner pressure value of the duct measured by the second pressure measuring unit (34b) at the setting time and a previously set target value of an inner pressure to the minimum.

## Patentansprüche

1. Endoskopballonsteuerungseinrichtung (7) mit einer ersten Pumpe (32a) zum Zuführen oder Ablassen von Gas zu oder von einem ersten Ballon (9) zum Befestigen des Endoskops, wobei der Ballon an ein peripheres Teil eines Kopfteils einer Einführungseinheit (2B) eines Endoskops (2) angebracht ist; und einer Steuerungseinheit (35) zum Steuern eines Drucks innerhalb des ersten Ballons (9) durch Betätigen der ersten Pumpe (32a) auf Grundlage des gemessenen Ergebnisses durch Messen eines Drucks eines Gases innerhalb des ersten Ballons (9), **dadurch gekennzeichnet, dass**
die Steuerungseinheit (35) eine Strömungsbetragerfassungsseinheit zum Erfassen des Strömungsbetrages des Gases aufweist, das in den ersten Ballon (9) gefördert oder von diesem abgezogen wird, und den Strömungsbetrag an Gas, das in den ersten Ballon (9) gefördert oder von diesem abgezogen wird, durch Betätigen der ersten Pumpe (32a) auf Grundlage des durch die Strömungsbetragerfassungseinheit erfassten Ergebnisses steuert.

2. Endoskopballonsteuerungseinrichtung nach Anspruch 1, **gekennzeichnet durch** Aufweisen einer zweiten Pumpe (23b) zum Zuführen oder Abgeben von Gas zu oder von einem zweiten Ballon (11) zum Befestigen des Endoskops, wobei der Ballon an einem peripheren Teil eines Kopfteils eines Überschlauches (3) für das Endoskop, das **dadurch** eingeführt wird, angebracht ist; wobei die Strömungsbetragerfassungseinheit dazu ausgebildet ist, den Strömungsbetrag des Gases, das in den ersten Ballon (9) und den zweiten Ballon des Überschlauches (3) gefördert wird oder davon abgezogen wird, zu erfassen; wobei die Steuerungseinheit (35) den Strömungsbetrag an Gas steuert, der in jeden von dem ersten und dem zweiten Ballon (9, 11) gefördert wird oder von diesem abgezogen wird, indem die zweite Pumpe (32b) auf Grundlage des **durch** die Strömungsbetragerfassungseinheit erfassten Ergebnisses betätigt wird.

3. Endoskopballonsteuerungseinrichtung nach Anspruch 1, **dadurch gekennzeichnet, dass** die Steuerungseinheit (35) eine Betätigung der ersten Pumpe (32a) stoppt, wenn das von der Strömungsbetragerfassungseinheit erfasste Ergebnis einen vorbestimmten Schwellenwert überschreitet.

4. Endoskopballonsteuerungseinrichtung nach Anspruch 2, **dadurch gekennzeichnet, dass** die Steuerungseinheit (35) eine Betätigung der zweiten Pumpe (32b) stoppt, wenn das von der Strömungsbetragerfassungseinheit erfasste Ergebnis einen vorbestimmten Schwellenwert überschreitet.

5. Endoskopballonsteuerungseinrichtung nach Anspruch 1, **gekennzeichnet durch** Aufweisen einer Fernsteuerungseinrichtung (8), die eine Mehrzahl von Betätigungstasten (18a, 19a, 20a, 21a) und eine Notaustaste (23) zum Abgeben von Hinweisen an die Steuerungseinheit (35) aufweist; wobei die Steuerungseinheit (35) eine Betätigung der ersten Pumpe (32a) beendet, wenn die Notaustaste (23) der Fernsteuerungseinrichtung (8) nieder gedrückt wird.

6. Endoskopballonsteuerungseinrichtung nach Anspruch 2, **gekennzeichnet durch** Aufweisen einer Fernsteuerungseinrichtung (8), die eine Mehrzahl von Betätigungstasten (18b, 19b, 20b, 21b) und eine Notaustaste (23) zum Abgeben von Hinweisen an die Steuerungseinheit (35) aufweist; wobei die Steuerungseinheit (35) einen Betrieb der zweiten Pumpe (32b) beendet, wenn die Notaustaste (23) der Fernsteuerungseinrichtung (8) niedergedrückt wird.

7. Endoskopballonsteuerungseinrichtung nach Anspruch 1, wobei die erste Pumpe (32a) zum Zuführen oder Abgeben von Gas zu oder von einer Leitung, die mit dem ersten Ballon (9) verbunden ist, vorgesehen ist und ferner aufweist eine erste Druckmesseinheit (34a) zum Messen eines inneren Drucks des ersten Ballons (9) oder der Leitung; eine erste Strömungsbetrageinstellungseinheit (32c) zum Einstellen des Strömungsbetrages des Gases der ersten Pumpe (32a), das in die Leitung gefördert wird; eine erste Zeiterfassungseinheit zum Erfassen einer Gasförderzeit oder einer Gasabzugszeit zu oder von dem ersten Ballon (9); wobei die Steuerungseinheit (35) vorgesehen ist zum Einstellen des Drucks innerhalb des ersten Ballons (9) durch Betätigen der ersten Pumpe (32a) und Steuern des Strömungsbetrages des Gases, das in den ersten Ballon gefördert wird oder von diesem abgezogen wird, auf Grundlage eines durch die erste Druckmesseinheit (34a) gemessenen Ergebnisses und eines durch die Strömungsbetragerfassungseinheit erfassten Ergebnisses und eines durch die erste Zeiterfassungseinheit erfassten Ergebnisses; wobei die Steuerungseinheit (35) aufweist einen Abnormalitätsbestimmungsabschnitt zum Ändern des Strömungsbetrages eines Gases, das von der ersten Pumpe (32a) der Leitung zugeführt wird, indem die erste Strömungsmengeneinstelleinheit gesteuert wird, Zuführen von Gas zu der Leitung und Erlangen eines ersten inneren Druckwerts und eines zweiten inneren Druckwerts der Leitung, die durch die erste Druckmesseinheit (34a) zu einem zuvor festgelegen ersten Zeitpunkt und zu einem zuvor festgelegten zweiten Zeitpunkt gemessen wurden, und, falls eine Differenz zwischen dem ersten inneren Druckwert und dem zweiten inneren Druckwert einen vorbestimmten Schwellenwert überschreitet, Bestimmen, dass der erste Ballon (9) abnormal ist; und eine Abnormal-Zustand-Informationseinheit zum Informieren über die Abnormalität der Leitung auf Grundlage des von dem Abnormalitätsbestimmungsabschnitt bestimmten Ergebnisses.

8. Endoskopballonsteuerungseinrichtung nach Anspruch 2, wobei die zweite Pumpe (32b) zum Zuführen oder Ablassen von Gas zu oder von einer Leitung, die an den zweiten Ballon (11) angeschlossen ist, vorgesehen ist und ferner aufweist eine zweite Druckmesseinheit (34b) zum Messen eines inneren Drucks des zweiten Ballons (11) oder der Leitung; eine zweite Strömungsbetragseinstelleinheit (32d) zum Einstellen des Strömungsbetrages des Gases der zweiten Pumpe (32b), das in die Leitung gefördert wird; eine zweite Zeiterfassungseinheit zum Erfassen einer Gasförderzeit oder einer Gasabzugszeit zu oder von dem zweiten Ballon (11); wobei die Steuerungseinheit (35) vorgesehen ist zum Einstellen eines Drucks innerhalb des zweiten Ballons (11) durch Betätigen der zweiten Pumpe (32b) und Steuern des Strömungsbetrages des Gases, das in den zweiten Ballon (11) gefördert wird oder von diesem abgezogen wird, auf Grundlage eines durch die zweite Druckmesseinheit (34b) gemessenen Ergebnisses und eines durch die Strömungsbetragerfassungseinheit erfassten Ergebnisses und eines durch die zweite Zeiterfassungseinheit erfassten Ergebnisses; wobei die Steuerungseinheit (35) aufweist einen Abnormalitätsbestimmungsabschnitt zum Ändern des Strömungsbetrages des Gases, das von der zweiten Pumpe (32b) in die Leitung gefördert wird, indem die zweite Strömungsbetrageinstelleinheit gesteuert wird, Zuführen von Gas zur Leitung und Erlangen eines ersten inneren Druckwerts und eines zweiten inneren Druckwerts der Leitung, die durch die zweite Druckmesseinheit (34b) zu einem zuvor festgelegten ersten Zeitpunkt und einem zuvor festgelegten zweiten Zeitpunkt gemessen werden, und, falls eine Differenz zwischen dem ersten inneren Druckwert und dem zweiten inneren Druckwert einen zuvor festgelegten Schwellenwert überschreitet, Bestimmen, dass der zweite Ballon (11) abnormal ist; und eine Abnormal-Zustand-Informationseinheit zum Informieren über die Abnormalität der Leitung auf Grundlage des bestimmten Ergebnisses des Abnormalitätsbestimmungsabschnittes.

9. Endoskopballonsteuerungseinrichtung nach Anspruch 7, **dadurch gekennzeichnet, dass** der Abnormalitätsbestimmunsabschnitt den Strömungsbetrag des Gases der ersten Pumpe (32a), das in die Leitung gefördert wird, auf den minimalen Strömungsbetrag des geförderten Gases oder auf den maximalen Strömungsbetrag des geförderten Gases ändert, indem die erste Strömungsbetrageinstelleinheit gesteuert wird und Gas der Leitung zuführt und einen inneren Druckwert der Leitung erhält, der durch die erste Druckmesseinheit (34a) zu einem zuvor bestimmten Einstellzeitpunkt gemessen wird, und, falls der innere Druckwert der Leitung einen zuvor festgelegten ersten Schwellenwert überschreitet, wenn der Einstellzeitpunkt verstrichen ist, nachdem Gas in die Leitung durch den minimalen Strömungsbetrag von Gas, das durch die erste Pumpe (32a) gefördert wird, zugeführt wird oder, falls der innere Druckwert der Leitung nicht einen zuvor festgelegten zweiten Schwellenwert erreicht, wenn der Einstellzeitpunkt verstrichen ist, nachdem Gas der Leitung durch den maximalen Strömungsbetrag von Gas zugeführt wird, das durch die erste Pumpe (32a) gefördert wird, bestimmt, dass die erste Pumpe (32a) abnormal ist.

10. Endoskopballonsteuerungseinrichtung nach Anspruch 8, **dadurch gekennzeichnet, dass** der Abnormalitätsbestimmungsabschnitt den Strömungsbetrag von Gas der zweiten Pumpe (32b), das in die Leitung gefördert wird, auf den minimalen Strömungsbetrag von gefördertem Gas oder den maximalen Strömungsbetrag von gefördertem Gas ändert, indem die zweite Strömungsbetrageinstelleinheit gesteuert wird und Gas der Leitung zuführt und einen inneren Druckwert der Leitung erhält, der durch die zweite Druckmesseinheit (34b) bei einem zuvor festgelegten Einstellzeitpunkt gemessen wird und, falls der innere Druckwert der Leitung einen zuvor festgelegten ersten Schwellenwert überschreitet, wenn der Einstellzeitpunkt verstrichen ist, nachdem das Gas in die Leitung durch den minimalen Strömungsbetrag des durch die zweite Pumpe (32b) geförderten Gases zugeführt wird, oder falls der innere Druckwert der Leitung nicht einen zuvor festgelegten Schwellenwert erreicht, wenn der Einstellzeitpunkt verstrichen ist, nachdem das Gas der Leitung durch den maximalen Strömungsbetrag des durch die zweite Pumpe (32b) geförderten Gases zugeführt wird, bestimmt, dass die zweite Pumpe (32b) abnormal ist.

11. Endoskopballonsteuerungseinrichtung nach Anspruch 9, **dadurch gekennzeichnet, dass** der Abnormalitätsbestimmungsabschnitt eine Strömungsbetrageinstellung durchführt, um eine Differenz zwischen einem inneren Druckwert der Leitung, der durch die erste Druckmesseinheit (34a) zum Einstellzeitpunkt gemessen wird, und einem zuvor festgelegten Zielwert des inneren Druckes auf das Minimum bringt.

12. Endoskopballonsteuerungseinrichtung nach Anspruch 10, **dadurch gekennzeichnet, dass** der Abnormalitätsbestimmungsabschnitt eine Strömungsbetrageinstellung durchführt, um eine Differenz zwischen einem inneren Druckwert der Leitung, der durch die zweite Druckmesseinheit (34b) zum Einstellzeitpunkt gemessen wird, und einem zuvor festgelegten Zielwert eines inneren Drucks auf das Minimum bringt.

## Revendications

1. Dispositif de commande de ballon endoscopique (7) comprenant une première pompe (32a) destinée à délivrer ou à évacuer du gaz vers ou provenant d'un premier ballon (9) pour fixer l'endoscope, ledit ballon étant fixé à une partie périphérique d'une partie de pointe d'une unité d'insertion (2B) d'un endoscope (2) ; et une unité de commande (35) destinée à commander une pression à l'intérieur du premier ballon (9) en actionnant la première pompe (32a) sur la base du résultat mesuré en mesurant une pression d'un gaz à l'intérieur du premier ballon (9), **caractérisé en ce que**
l'unité de commande (35) comporte une unité de détection de quantité d'écoulement destinée à détecter la quantité d'écoulement de gaz envoyé ou aspiré vers ou depuis le premier ballon (9), et commande la quantité d'écoulement de gaz envoyé ou aspiré vers ou depuis le premier ballon (9) en actionnant la première pompe (32a) sur la base du résultat détecté par l'unité de détection de quantité d'écoulement.

2. Dispositif de commande de ballon endoscopique selon la revendication 1, **caractérisé en ce qu'**il comprend une deuxième pompe (32b) destinée à délivrer ou à évacuer un gaz vers ou depuis un deuxième ballon (11) pour fixer l'endoscope, ledit ballon étant fixé à une partie périphérique d'une partie de pointe d'une gaine (3) pour l'endoscope (2) étant inséré à travers celle-ci ; l'unité de détection de quantité d'écoulement étant adaptée pour détecter la quantité d'écoulement de gaz envoyé ou aspiré vers ou depuis le premier ballon (9) et le deuxième ballon de la gaine (3) ; dans lequel l'unité de commande (35) commande la quantité d'écoulement de gaz envoyé ou aspiré vers ou depuis chacun des premier et deuxième ballons (9, 11) en actionnant la deuxième pompe (32b) sur la base du résultat détecté par l'unité de détection de quantité d'écoulement.

3. Dispositif de commande de ballon endoscopique selon la revendication 1, **caractérisé en ce que** l'unité de commande (35) arrête un fonctionnement de la première pompe (32a) lorsque le résultat détecté de l'unité de détection de quantité d'écoulement dépasse un seuil prédéterminé.

4. Dispositif de commande de ballon endoscopique selon la revendication 1, **caractérisé en ce que** l'unité de commande (35) arrête un fonctionnement de la deuxième pompe (32b) lorsque le résultat détecté de l'unité de détection de quantité d'écoulement dépasse un seuil prédéterminé.

5. Dispositif de commande de ballon endoscopique selon la revendication 1, **caractérisé en ce qu'**il comprend un contrôleur à distance (8) comprenant une pluralité de boutons de manipulation (18a, 19a, 20a, 21a) et un bouton d'arrêt d'urgence (23) destiné à donner des indications à l'unité de commande (35) ; dans lequel l'unité de commande (35) arrête un fonctionnement de la première pompe (32a) lorsque le bouton d'arrêt d'urgence (23) du contrôleur à distance (8) est enfoncé.

6. Dispositif de commande de ballon endoscopique selon la revendication 2, **caractérisé en ce qu'**il comprend un contrôleur à distance (8) comprenant une pluralité de boutons de manipulation (18b, 19b, 20b, 21b) et un bouton d'arrêt d'urgence (23) destiné à donner des indications à l'unité de commande (35) ; dans lequel l'unité de commande (35) arrête un fonctionnement de la deuxième pompe (32b) lorsque le bouton d'arrêt d'urgence (23) du contrôleur à distance (8) est enfoncé.

7. Dispositif de commande de ballon endoscopique selon la revendication 1, dans lequel la première pompe (32a) est destinée à délivrer ou à évacuer un gaz vers ou depuis un conduit raccordé au premier ballon (9) et comprend en outre une première unité de mesure de pression (34a) destinée à mesurer une pression interne du premier ballon (9) ou du conduit ; une première unité d'ajustement de quantité d'écoulement (32c) destinée à ajuster la quantité d'écoulement de gaz de la première pompe (32a) envoyé vers le conduit ; une première unité de détection de temps destinée à détecter un temps d'envoi de gaz ou un temps d'aspiration de gaz vers ou depuis le premier ballon (9) ; dans lequel l'unité de commande (35) est destinée à ajuster la pression à l'intérieur du premier ballon (9) en actionnant la première pompe (32a) et en commandant la quantité d'écoulement de gaz envoyé ou aspiré vers ou depuis le premier ballon (9) sur la base d'un résultat mesuré par la première unité de mesure de pression (34a) et d'un résultat détecté par l'unité de détection de quantité d'écoulement et d'un résultat détecté par la première unité de détection de temps ; dans lequel l'unité de commande (35) comprend une section de détermination d'anomalie destinée à changer la quantité d'écoulement de gaz envoyé depuis la première pompe (32a) vers le conduit en commandant la première unité d'ajustement de quantité d'écoulement, délivrer le gaz vers le conduit et obtenir une première valeur de pression interne et une deuxième valeur de pression interne du conduit mesurées par la première unité de mesure de pression (34a) à un premier temps préalablement établi et à un deuxième temps préalablement établi et, si une différence entre la première valeur de pression interne et la deuxième valeur de pression interne dépasse un seuil préalablement établi, déterminer que le premier ballon (9) est anormal ; et une unité d'information d'état d'anomalie destinée à informer de l'anomalie du conduit sur la base du résultat déterminé de la section de détermination d'anomalie.

8. Dispositif de commande de ballon endoscopique selon la revendication 2, dans lequel la deuxième pompe (32b) est destiné à délivrer ou à évacuer un gaz vers ou depuis un conduit raccordé au deuxième ballon (11) et comprend en outre une deuxième unité de mesure de pression (34b) destinée à mesurer une pression interne du deuxième ballon (11) ou du conduit ; une deuxième unité d'ajustement de quantité d'écoulement (32d) destinée à ajuster la quantité d'écoulement de gaz de la deuxième pompe (32b) envoyé vers le conduit ; une deuxième unité de détection de temps destinée à détecter un temps d'envoi de gaz ou un temps d'aspiration de gaz vers ou depuis le deuxième ballon (11) ; dans lequel l'unité de commande (35) est destinée à ajuster la pression à l'intérieur du deuxième ballon (11) en actionnant la deuxième pompe (32b) et en commandant la quantité d'écoulement de gaz envoyé ou aspiré vers ou depuis le deuxième ballon (11) sur la base d'un résultat mesuré par la deuxième unité de mesure de pression (34b) et d'un résultat détecté par l'unité de détection de quantité d'écoulement et d'un résultat détecté par la deuxième unité de détection de temps ; dans lequel l'unité de commande (35) comprend une section de détermination d'anomalie destinée à changer la quantité d'écoulement de gaz envoyé depuis la deuxième pompe (32b) vers le conduit en commandant la deuxième unité d'ajustement de quantité d'écoulement, délivrer le gaz vers le conduit et obtenir une première valeur de pression interne et une deuxième valeur de pression interne du conduit mesurées par la deuxième unité de mesure de pression (34b) à un premier temps préalablement établi et à un deuxième temps préalablement établi et, si une différence entre la première valeur de pression interne et la deuxième valeur de pression interne dépasse un seuil préalablement établi, déterminer que le deuxième ballon (11) est anormal ; et une unité d'information d'état d'anomalie destinée à informer de l'anomalie du conduit sur la base du résultat déterminé de la section de détermination d'anomalie.

9. Dispositif de commande de ballon endoscopique selon la revendication 7, **caractérisé en ce que** la section de détermination d'anomalie change la quantité d'écoulement de gaz de la première pompe (32a) envoyé vers le conduit à la quantité minimale d'écoulement de gaz envoyé ou à la quantité maximale d'écoulement de gaz envoyé en commandant la première unité d'ajustement de quantité d'écoulement et délivre le gaz vers le conduit et obtient une valeur de pression interne du conduit mesurée par la première unité de mesure de pression (34a) à un temps d'établissement préalablement établi, et si la valeur de pression interne du conduit dépasse un premier seuil préalablement établi lorsque le temps d'établissement a passé après que le gaz ait été délivré vers le conduit de la quantité minimale d'écoulement de gaz envoyé par la première pompe (32a), ou si la valeur de pression interne du conduit n'atteint pas un deuxième seuil préalablement établi lorsque le temps d'établissement a passé après que le gaz ait été délivré vers le conduit de la quantité maximale d'écoulement de gaz envoyé par la première pompe (32a), détermine que la première pompe (32a) est anormale.

10. Dispositif de commande de ballon endoscopique selon la revendication 8, **caractérisé en ce que** la section de détermination d'anomalie change la quantité d'écoulement de gaz de la deuxième pompe (32b) envoyé vers le conduit à la quantité minimale d'écoulement de gaz envoyé ou à la quantité maximale d'écoulement de gaz envoyé en commandant la deuxième unité d'ajustement de quantité d'écoulement et délivre le gaz vers le conduit et obtient une valeur de pression interne du conduit mesurée par la deuxième unité de mesure de pression (34b) à un temps d'établissement préalablement établi, et si la valeur de pression interne du conduit dépasse un premier seuil préalablement établi lorsque le temps d'établissement a passé après que le gaz ait été délivré vers le conduit de la quantité minimale d'écoulement de gaz envoyé par la deuxième pompe (32b), ou si la valeur de pression interne du conduit n'atteint pas un deuxième seuil préalablement établi lorsque le temps d'établissement a passé après que le gaz ait été délivré vers le conduit de la quantité maximale d'écoulement de gaz envoyé par la deuxième pompe (32b), détermine que la deuxième pompe (32b) est anormale.

11. Dispositif de commande de ballon endoscopique selon la revendication 9, **caractérisé en ce que** la section de détermination d'anomalie réalise un ajustement de la quantité d'écoulement pour faire une différence entre une valeur de pression interne du conduit mesurée par la première unité de mesure de pression (34a) au temps d'établissement et une valeur cible préalablement établie d'une pression interne au minimum.

12. Dispositif de commande de ballon endoscopique selon la revendication 10, **caractérisé en ce que** la section de détermination d'anomalie réalise un ajustement de la quantité d'écoulement pour faire une différence entre une valeur de pression interne du conduit mesurée par la deuxième unité de mesure de pression (34b) au temps d'établissement et une valeur cible préalablement établie d'une pression interne au minimum.
